# EUROPEAN PATENT SPECIFICATION

(11) **EP 2 421 509 B1**
(45) Date of publication and mention of the grant of the patent: **22.11.2017**
(21) Application number: 10766513.5
(22) Date of filing: 23.04.2010
(51) Int. Cl.: A61K 9/14

(54) **PRODUCTION OF ENCAPSULATED NANOPARTICLES AT HIGH VOLUME FRACTIONS**
HERSTELLUNG VON VERKAPSELTEN NANOPARTIKELN MIT HOHEN VOLUMENANTEILEN
PRODUCTION DE NANOPARTICULES ENCAPSULÉES À DES FRACTIONS VOLUMIQUES ÉLEVÉES

(30) Priority: 24.04.2009 AU 2009901744; 24.04.2009 US 172278 P
(43) Date of publication of application: 29.02.2012
(73) Proprietor: Iceutica Pty Ltd., Mouth Hawthorn, WA 6016 (AU)
(72) Inventor: DODD, Aaron, Centennial Park New South Wales 2021 (AU); MEISER, Felix, Mount Claremont Western Australia 6010 (AU); RUSSELL, Adrian, Rivervale Western Australia 6103 (AU); NORRET, Marck, Darlington Western Australia 6070 (AU); BOSCH, H., William, Bryn Mawr Pennsylvania 19010 (US)
(74) Representative: Conroy, John
(86) International application number: PCT/AU2010/000464
(87) International publication number: WO 2010/121320

(56) References cited:
- EP-A1- 0 600 528
- WO-A1-2006/031026
- WO-A1-2008/000042
- WO-A1-2010/017104
- WO-A2-2008/118331
- US-A1- 2004 037 785
- US-A1- 2008 254 128
- BARZEGAR-JALALI, M. ET AL.: 'Evaluation of in vitro - in vivo correlation and anticonvulsive effect of carbamazepine after cogrinding with microcrystalline cellulose' J PHARM PHARMACEUT SCIENCE vol. 9, no. 3, 2006, pages 307 - 316, XP008154546

## Description

### Field of the Invention

The present invention relates to methods for producing particles of a biologically active material using dry milling processes.

### Background

Poor bioavailability is a significant problem encountered in the development of compositions in the therapeutic, cosmetic, agricultural and food industries, particularly those materials containing a biologically active material that is poorly soluble in water at physiological pH. An active material's bioavailability is the degree to which the active material becomes available to the target tissue in the body or other medium after systemic administration through, for example, oral or intravenous means. Many factors affect bioavailability, including the form of dosage and the solubility and dissolution rate of the active material.

In therapeutic applications, poorly and slowly water-soluble materials tend to be eliminated from the gastrointestinal tract before being absorbed into the circulation. In addition, poorly soluble active agents tend to be disfavored or even unsafe for intravenous administration due to the risk of particles of agent blocking blood flow through capillaries.

It is known that the rate of dissolution of a particulate drug will increase with increasing surface area. One way of increasing surface area is decreasing particle size. Consequently, methods of making finely divided or sized drugs have been studied with a view to controlling the size and size range of drug particles for pharmaceutical compositions.

For example, dry milling techniques have been used to reduce particle size and hence influence drug absorption. However, in conventional dry milling the limit of fineness is reached generally in the region of about 100 microns (100,000 nm), at which point material cakes on the milling chamber and prevents any further diminution of particle size. Alternatively, wet grinding may be employed to reduce particle size, but flocculation restricts the lower particle size limit to approximately 10 microns (10,000 nm). The wet milling process, however, is prone to contamination, thereby leading to a bias in the pharmaceutical art against wet milling. Another alternative milling technique, commercial airjet milling, has provided particles ranging in average size from as low as about 1 to about 50 microns (1,000-50,000 nm).

There are several approaches currently used to formulate poorly soluble active agents. One approach is to prepare the active agent as a soluble salt. Where this approach cannot be employed, alternate (usually physical) approaches are employed to improve the solubility of the active agent. Alternate approaches generally subject the active agent to physical conditions that change the agent's physical and or chemical properties to improve its solubility. These include process technologies such as micro-ionisation, modification of crystal or polymorphic structure, development of oil based solutions, use of co-solvents, surface stabilizers or complexing agents, micro-emulsions, super critical fluid and production of solid dispersions or solutions. More than one of these processes may be used in combination to improve formulation of a particular therapeutic material. Many of these approaches commonly convert a drug into an amorphous state, which generally leads to a higher dissolution rate. However, formulation approaches that result in the production of amorphous material are not common in commercial formulations due to concerns relating to stability and the potential for material to re-crystallize.

These techniques for preparing such pharmaceutical compositions tend to be complex. By way of example, a principal technical difficulty encountered with emulsion polymerization is the removal of contaminants, such as unreacted monomers or initiators (which may have undesirable levels of toxicity), at the end of the manufacturing process.

Another method of providing reduced particle size is the formation of pharmaceutical drug microcapsules, which techniques include micronizing, polymerisation and co-dispersion. However, these techniques suffer from a number of disadvantages including at least the inability to produce sufficiently small particles such as those obtained by milling, and the presence of co-solvents and/or contaminants such as toxic monomers which are difficult to remove, leading to expensive manufacturing processes.

Over the last decade, intense scientific investigation has been carried out to improve the solubility of active agents by converting the agents to ultra fine powders by methods such as milling and grinding. These techniques may be used to increase the dissolution rate of a particulate solid by increasing the overall surface area and decreasing the mean particle size.

US Patent 6,634,576 discloses examples of wet-milling a solid substrate, such as a pharmaceutically active compound, to produce a "synergetic co-mixture".

International Patent Application PCT/AU2005/001977 (Nanoparticle Composition(s) and Method for Synthesis Thereof) describes, *inter alia,* a method comprising the step of contacting a precursor compound with a co-reactant under mechanochemical synthesis conditions wherein a solid-state chemical reaction between the precursor compound and the co-reactant produces therapeutically active nanoparticles dispersed in a carrier matrix. Mechanochemical synthesis, as discussed in International Patent Application PCT/AU2005/001977, refers to the use of mechanical energy to activate, initiate or promote a chemical reaction, a crystal structure transformation or a phase change in a material or a mixture of materials, for example by agitating a reaction mixture in the presence of a milling media to transfer mechanical energy to the reaction mixture, and includes without limitation "mechanochemical activation", "mechanochemical processing", "reactive milling", and related processes.

International Patent Application PCT/AU2007/000910 (Methods for the preparation of biologically active compounds in nanoparticulate form) describes, *inter alia,* a method for dry milling raloxifene with lactose and NaCl which produced nanoparticulate raloxifene without significant aggregation problems. The methods disclosed by the prior art produce nanoparticles at volume fractions of 15% or less and suggests that 25% is the upper limit for the volume fraction of the biologically active material that could be successfully converted to smaller particles.

The present invention provides methods for an improved milling process which produces particles of active compound with increased surface area, yet allows for higher volume fractions of the biologically active material.

One example of a therapeutic area where this technology could be applied in is the area of acute pain management. Many pain medications such as naproxen provides pain relief for chronic pain. As a result they are commonly taken on a daily basis to maintain an effective therapeutic level. Because naproxen is a poorly water soluble drug dissolution and absorbtion to the body is slow with the Tmax of current commercial formulations in the range of 1-4 hours. So a method such as the present invention which provides for improved dissolution, will likely provide much faster absorption resulting in a more rapid onset of the therapeutic effect. By using a method such as the present invention, which provides faster absorption, a drug such as naproxen, could be used more readily to treat acute pain as well as chronic pain.

Naproxen dosages typically range from 200-500 mg of active. Because of this requirement for high amounts of active ingredient pervious art which produced nanoparticles at 15% would be difficult to use to produce a commercial formulation. As the present invention provides for the production of particles at higher volume fractions is it more suitable for medications such as naproxen.

Another example is the drug metaxalone which is commercially marketed under the name Skelaxin®. Skelaxin® is indicated as an adjunct to rest, physical therapy, and other measures for the relief of discomforts associated with acute, painful musculoskeletal conditions and is taken as an 800 mg tablet three to four times a day. Previous animal studies have shown that by reducing the size of metaxalone much higher rates of absortion and overall bioavaiability (as measured by AUC) can be achieved. The present invention being able to produce small particles (with increased surface area) at high volume volume fractions is thus well suited to a drug such as metaxalone. So a method such as the present invention which provides for improved dissolution and potentially higher bioavailability could result in a formulation of metaxalone where much less active is required to deliver the same therapeutic effect.

Although the background to the present invention is discussed in the context of improving the bioavailability of materials that are poorly or slowly water soluble, the applications of the methods of the present invention are not limited to such, as is evident from the following description of the invention.

Further, although the background to the present invention is largely discussed in the context of improving the bioavailability of therapeutic or pharmaceutical compounds, the applications of the methods of the present invention are clearly not limited to such. For example, as is evident from the following description, applications of the methods of the present invention include but are not limited to: neutraceutical and nutritional compounds, complementary medicinal compounds, veterinary therapeutic applications and agricultural chemical applications, such as pesticide, fungicide or herbicide.

Furthermore an application of the method disclosed herein would be to materials which contain a biologically active compound such as, but not limited to a therapeutic or pharmaceutical compound, a neutraceutical or nutrient, a complementary medicinal product such as active components in plant or other naturally occurring material, a veterinary therapeutic compound or an agricultural compound such as a pesticide, fungicide or herbicide. Specific examples would be the spice turmeric that contains the active compound curcumin, or flax seed that contains the nutrient ALA an omega 3 fatty acid. As these specific examples indicate the method disclosed herein could be applied to, but not limited to, a range of natural products such as seeds, cocoa and cocoa solids, coffee, herbs, spices, other plant materials or food materials that contain a biologically active compound. The application of the method disclosed herein to these types of materials would enable greater availability of the active compound in the materials when used in the relevant application. For example where material is orally ingested the active would be more bioavailable.

US 2004/037785 A1 relates to a method for making particles for use in a pharmaceutical composition. EP 0 600 528 A1 relates to a method for ginding pharmaceutical substances. Barzegar-Jalali, M. et al. describe the evaluation of the in vitro/in vivo correlation and the anticonvulsive effect of carbamazepine after cogrinding with microcrystalline cellulose (J Pharm Pharmaceut Science, vol. 9, no. 3, 2006, pages 307-316), US 2008/0245128 A1 relates to a process for the precipitation and isolation of 6,6-dimethyl-3-aza-bicyclo [3.1.0] hexane-amide compounds by controlled precipitation and pharmaceutical formulations containinin the same. WO 2008/118331 A2 relates to tablet formulations containing 8-[{1-(3,5-Bis-(trifluoromethyl)phenyl)-ethoxy}-methyl]-8-phenyl-1,7-diaza-spiro[4.5]decan-2-one salts and tablets made therefrom.

### Summary of the Invention

The invention is directed to a method for producing a composition, comprising the steps of:
dry milling a solid biologically active material, a milling aid or combination of milling aids, and a millable grinding matrix in a mill comprising a plurality of milling bodies, for a time period sufficient to produce particles of the biologically active material dispersed in an at least partially milled grinding material, wherein the composition produced by said method comprises particles of the biologically active compound at or above a volume fraction of 25 v/v%, wherein the particles have a median particle size, determined on a particle volume basis, equal or less than 800 nm.

The particles can have a median particle size, determined on a particle volume basis, equal or less than a size selected from the group consisting of: 700nm, 600nm, 500nm, 400 nm, 300nm, 200nm and 100 nm.

The milling aid can be selected from the group consisting of: colloidal silica, a solid or semi solid surfactant, a liquid surfactant, a surfactant that can be manufactured into a solid or semisolid, a polymer, a stearic acid and derivatives thereof.

The surfactant can be selected from the group consisting of: polyoxyethylene alkyl ethers, polyoxyethylene stearates, poloxamers, sarcosine based surfactants, polysorbates, alkyl sulfates and other sulfate surfactants, ethoxylated castor oil, polyvinylpyrrolidones, deoxycholate based surfactants, trimethyl ammonium based surfactants, lecithin and other phospholipids and bile salts.

The milling aid can be sodium lauryl sulfate. The millable grinding matrix can be lactose anhydrous or lactose monohydrate. The millable grinding matrix can be is mannitol.

### DISCLOSURE

The unexpected finding is disclosed that particles of a biologically active material can be produced by dry milling processes wherein the composition produced by said method comprises particles of the biologically active material at or above a volume fraction of 25 v/v%. In one surprising aspect the particle size produced by the process is equal to or less than 2000nm. In another surprising aspect the particle size produced by the process is equal to or less than 1000nm. In another surprising aspect the crystallinity of the active material is unchanged or not substantially changed.

The method can comprise particles of the biologically active material at or above a volume fraction selected from the group consisting of 25 v/v%; 30 v/v%; 35 v/v%; 40 v/v%; 45 v/v%; 50 v/v%, 55 v/v% and 60 v/v%. The method can comprise particles of the biologically active material at or below a volume fraction selected from the group consisting of 60 v/v%, 55 v/v%, 50 v/v%; 45 v/v%; 40 v/v%; and 35 v/v%.

Disclosed is a method producing a composition, comprising the steps of dry milling a solid biologically active material and a millable grinding matrix in a mill comprising a plurality of milling bodies, for a time period sufficient to produce particles of the biologically active material dispersed in an at least partially milled grinding material, wherein the composition produced by said method comprises particles of the biologically active material at or above a volume fraction of 25 v/v%.

The average particle size, determined on a particle number basis, can be equal to or less than a size selected from the group 10,000nm, 8000nm, 6000nm, 5000nm, 4000nm, 3000nm, 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The average particle size can be equal to or greater than 25nm.

The particles can have a median particle size, determined on a particle volume basis, equal or less than a size selected from the group 20000nm, 15000nm, 10000 nm, 7500nm, 5000nm, 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The median particle size can be equal to or greater than 25nm. The percentage of particles, on a particle volume basis, can be selected from the group consisting of: 50%, 60%, 70%, 80%, 90%, 95% and 100 % less than 2000nm (% < 2000 nm). The percentage of particles, on a particle volume basis, can be selected from the group consisting of: 50%, 60%, 70%, 80%, 90%, 95% and 100 % less than 1000nm (% < 1000 nm). The percentage of particles, on a particle volume basis, can be selected from the group 0%, 10%, 20%, 30%, 40%, 50%, 60%, 70%, 80%, 90%, 95% and 100 % less than 500nm (% < 500 nm). The percentage of particles, on a particle volume basis, can be selected from the group 0%, 10%, 20%, 30%, 40%, 50%, 60%, 70%, 80%, 90%, 95% and 100 % less than 300nm (% < 300 nm). The percentage of particles, on a particle volume basis, can be selected from the group 0%, 10%, 20%, 30%, 40%, 50%, 60%, 70%, 80%, 90%, 95% and 100 % less than 200nm (% < 200 nm). The Dx of the particle size distribution, as measured on a particle volume basis, can be selected from the group consisting of less than or equal to 10,000nm, 5000nm, 3000nm, 2000nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm, and 100 nm; wherein x is greater than or equal to 90

The crystallinity profile of the biologically active material can be selected from the group consisting of: at least 50% of the biologically active material is crystalline, at least 60% of the biologically active material is crystalline, at least 70% of the biologically active material is crystalline, at least 75% of the biologically active material is crystalline, at least 85% of the biologically active material is crystalline, at least 90% of the biologically active material is crystalline, at least 95% of the biologically active material is crystalline and at least 98% of the biologically active material is crystalline. The crystallinity profile of the biologically active material can be substantially equal to the crystallinity profile of the biologically active material before the material was subjected to the method as described herein.

The amorphous content of the biologically active material can be selected from the group consisting of: less than 50% of the biologically active material is amorphous, less than 40% of the biologically active material is amorphous, less than 30% of the biologically active material is amorphous, less than 25% of the biologically active material is amorphous, less than 15% of the biologically active material is amorphous, less than 10% of the biologically active material is amorphous, less than 5% of the biologically active material is amorphous and less than 2% of the biologically active material is amorphous. The biologically active material can have no significant increase in amorphous content before subjecting the material to the method as described herein.

The milling time period can be a range selected from the group consisting of: between 10 minutes and 2 hours, between 10 minutes and 90 minutes, between 10 minutes and 1 hour, between 10 minutes and 45 minutes, between 10 minutes and 30 minutes, between 5 minutes and 30 minutes, between 5 minutes and 20 minutes, between 2 minutes and 10 minutes, between 2 minutes and 5 minutes, between 1 minutes and 20 minutes, between 1 minute and 10 minutes, and between 1 minute and 5 minutes.

The milling medium can be selected from the group consisting of: ceramics, glasses, polymers, ferromagnetics and metals. The milling medium can be steel balls having a diameter selected from the group consisting of: between 1 and 20 mm, between 2 and 15 mm and between 3 and 10 mm. The dry milling apparatus can be a mill selected from the group consisting of: attritor mills (horizontal or vertical), nutating mills, tower mills, pearl mills, planetary mills, vibratory mills, eccentric vibratory mills, gravity-dependent-type ball mills, rod mills, roller mills and crusher mills. The milling medium within the milling apparatus can be mechanical agitated by 1, 2 or 3 rotating shafts. The method can be configured to produce the biologically active material in a continuous fashion.

The total combined amount of biologically active material and grinding matrix in the mill at any given time can be equal to or greater than a mass selected from the group consisting of: 200 grams, 500 grams, 1 kg, 2kg, 5kg, 10kg, 20kg, 30kg, 50kg, 75kg, 100kg, 150kg, 200kg. The total combined amount of biologically active material and grinding matrix can be less than 2000kg. The biologically active material can be selected from the group consisting of: fungicides, pesticides, herbicides, seed treatments, cosmeceuticals, cosmetics, complementary medicines, natural products, vitamins, nutrients, neutraceuticals, pharmaceutical actives, biologics, amino acids, proteins, peptides, nucleotides, nucleic acids, additives, foods and food ingredients and analogs, homologs and first order derivatives thereof. The biologically active material can be selected from the group consisting of: anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, cough suppressants (expectorants and mucolytics), diagnostic agents, diagnostic imaging agents, diuretics, dopaminergics (anti-parkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines. The biologically active material can be selected from the group consisting of: indomethacin, diclofenac, naproxen, meloxicam, metaxalone, cyclosporin A, progesterone celecoxib, cilostazol, ciprofloxacin, 2,4-dichlorophenoxyacetic acid, anthraquinone, creatine monohydrate, glyphosate, halusulfuron, mancozeb, metsulfuron, salbutamol, sulphur, tribenuran and estradiol or any salt or derivative thereof.

The grinding matrix can be a single material or is a mixture of two or more materials in any proportion. The single material or a mixture of two or more materials can be selected from the group consisting of: mannitol, sorbitol, Isomalt, xylitol, maltitol, lactitol, erythritol, arabitol, ribitol, glucose, fructose, mannose, galactose, anhydrous lactose, lactose monohydrate, sucrose, maltose, trehalose, maltodextrins, dextrin, Inulin, dextrates, polydextrose, starch, wheat flour, corn flour, rice flour, rice starch, tapioca flour, tapioca starch, potato flour, potato starch, other flours and starches, milk powder, skim milk powders, other milk solids and dreviatives, soy flour, soy meal or other soy products, cellulose, microcystalline cellulose, microcystalline cellulose based co blended materials, pregelatinized (or partially) starch, HPMC, CMC, HPC, citric acid, tartaric acid, malic acid, maleic acid fumaric acid , ascorbic acid, succinic acid, sodium citrate, sodium tartrate, sodium malate, sodium ascorbate, potassium citrate, potassium tartrate, potassium malate, potassium ascorbate, sodium carbonate, potassium carbonate, magnesium carbonate, sodium bicarbonate, potassium bicarbonate and calcium carbonate. dibasic calcium phosphate, tribasic calcium phosphate, sodium sulfate, sodium chloride, sodium metabisulphite, sodium thiosulfate, ammonium chloride, Glauber's salt, ammonium carbonate, sodium bisulfate, magnesium sulfate, potash alum, potassium chloride, sodium hydrogen sulfate, sodium hydroxide, crystalline hydroxides, hydrogen carbonates, ammonium chloride, methylamine hydrochloride, ammonium bromide, silica, thermal silica, alumina, titanium dioxide, talc, chalk, mica, kaolin, bentonite, hectorite, magnesium trisilicate, clay based materials or aluminium silicates, sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, poloxamer 407, poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines. The concentration of the single (or first) material can be selected from the group consisting of: 5 - 99 % w/w, 10 - 95 % w/w, 15 - 85 % w/w, of 20 - 80% w/w, 25 - 75 % w/w, 30 - 60% w/w, 40 -50% w/w. ably, the concentration of the second or subsequent material is selected from the group consisting of: 5 - 50 % w/w, 5 - 40 % w/w, 5 - 30 % w/w, of 5 - 20% w/w, 10 - 40 % w/w, 10 -30% w/w, 10 -20% w/w, 20 - 40% w/w, or 20 - 30% w/w or if the second or subsequent material is a surfactant or water soluble polymer the concentration is selected from 0.1 -10 % w/w, 0.1 -5 % w/w, 0.1 -2.5 % w/w, of 0.1 - 2% w/w, 0.1 -1 %, 0.5 -5% w/w, 0.5 -3% w/w, 0.5 -2% w/w, 0.5 - 1.5%, 0.5 -1 % w/w, of 0.75 - 1.25 % w/w, 0.75-1% and 1% w/w.

The grinding matrix can be selected from the group consisting of:
(a) lactose anhydrous or lactose anhydrous combined with at least one material selected from the group consisting of: lactose monohydrate; xylitol; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(b) mannitol or mannitol combined with at least one material selected from the group consisting of: lactose monohydrate; xylitol; lactose anhydrous; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(c) Sucrose or sucrose combined with at least one material selected from the group consisting of: lactose monohydrate; lactose anhydrous; mannitol; microcrystalline cellulose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(d) Glucose or glucose combined with at least one material selected from the group consisting of: lactose monohydrate; lactose anhydrous; mannitol; microcrystalline cellulose; sucrose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(e) Sodium chloride or sodium chloride combined with at least one material selected from the group consisting of: lactose monohydrate; lactose anhydrous; mannitol; microcrystalline cellulose; sucrose; glucose; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(f) xylitol or xylitol combined with at least one material selected from the group consisting of: lactose monohydrate; lactose anhydrous; mannitol; microcrystalline cellulose; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(g) Tartaric acid or tartaric acid combined with at least one material selected from the group consisting of: lactose monohydrate; lactose anhydrous; mannitol; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(h) microcrystalline cellulose or microcrystalline cellulose combined with at least one material selected from the group consisting of: lactose monohydrate; xylitol; lactose anhydrous; mannitol; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(i) Kaolin combined with at least one material selected from the group consisting of: lactose monohydrate; xylitol; lactose anhydrous; mannitol; microcrystalline cellulose; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.
(j) Talc combined with at least one material selected from the group consisting of: lactose monohydrate; xylitol; lactose anhydrous; mannitol; microcrystalline cellulose; sucrose; glucose; sodium chloride; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone;; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

The grinding matrix can be selected from the group consisting of: a material considered to be Generally Regarded as Safe (GRAS) for pharmaceutical products; a material considered acceptable for use in an agricultural formulation; and a material considered acceptable for use in a veterinary formulation.

A milling aid or combination of milling aids can also be used. The milling aid can be selected from the group consisting of: colloidal silica, a surfactant, a polymer, a stearic acid and derivatives thereof. The surfactant can be in a solid form or can be manufactured into a solid form. The surfactant can be selected from the group consisting of: polyoxyethylene alkyl ethers, polyoxyethylene stearates, polyethylene glycols (PEG), poloxamers, poloxamines, sarcosine based surfactants, polysorbates, aliphatic alcohols, alkyl and aryl sulfates, alkyl and aryl polyether sulfonates and other sulfate surfactants, trimethyl ammonium based surfactants, lecithin and other phospholipids, bile salts, polyoxyethylene castor oil derivatives, polyoxyethylene sorbitan fatty acid esters, Sorbitan fatty acid esters, Sucrose fatty acid esters, alkyl glucopyranosides, alkyl maltopyranosides, glycerol fatty acid esters, Alkyl Benzene Sulphonic Acids, Alkyl Ether Carboxylic Acids, Alkyl and aryl Phosphate esters, Alkyl and aryl Sulphate esters, Alkyl and aryl Sulphonic acids, Alkyl Phenol Phosphates esters, Alkyl Phenol Sulphates esters, Alkyl and Aryl Phosphates, Alkyl Polysaccharides, Alkylamine Ethoxylates, Alkyl-Naphthalene Sulphonates formaldehyde condensates, Sulfosuccinates, lignosulfonates, Ceto-Oleyl Alcohol Ethoxylates, Condensed Naphthalene Sulphonates, Dialkyl and Alkyl Naphthalene Sulphonates,Di-alkyl Sulphosuccinates, Ethoxylated nonylphenols, Ethylene Glycol Esters, Fatty Alcohol Alkoxylates, Hydrogenated tallowalkylamines, Mono-alkyl Sulphosuccinamates, Nonyl Phenol Ethoxylates, Sodium Oleyl N-methyl Taurate, Tallowalkylamines, linear and branched dodecylbenzene sulfonic acids.

The surfactant can be selected from the group consisting of: sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, poloxamer 407, poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines. The polymer can be selected from the list of: polyvinylpyrrolidones (PVP), polyvinylalcohol, Acrylic acid based polymers and copolymers of acrylic acid

The milling aid can have a concentration selected from the group consisting of: 0.1 -10 % w/w, 0.1 -5% w/w, 0.1 -2.5% w/w, of 0.1 - 2% w/w, 0.1 -1 %, 0.5 -5% w/w, 0.5 -3% w/w, 0.5 -2% w/w, 0.5 - 1.5%, 0.5 -1 % w/w, of 0.75 - 1.25% w/w, 0.75 -1% and 1% w/w.

A facilitating agent can also be used or combination of facilitating agents can be used. The facilitating agent can be selected from the group consisting of: surfactants, polymers, binding agents, filling agents, lubricating agents, sweeteners, flavouring agents, preservatives, buffers, wetting agents, disintegrants, effervescent agents, agents that may form part of a medicament, including a solid dosage form or a dry powder inhalation formulation and other material required for specific drug delivery. The facilitating agent can be added during dry milling. The facilitating agent can be added to the dry milling at a time selected from the group consisting of: with 1-5 % of the total milling time remaining, with 1-10 % of the total milling time remaining, with 1-20 % of the total milling time remaining, with 1-30 % of the total milling time remaining, with 2-5% of the total milling time remaining, with 2-10% of the total milling time remaining, with 5-20% of the total milling time remaining and with 5-20% of the total milling time remaining. The disintegrant can be selected from the group consisting of: crosslinked PVP, cross linked carmellose and sodium starch glycolate. The facilitating agent can be added to the milled biologically active material and grinding matrix and further processed in a mechanofusion process. Mechanofusion milling causes mechanical energy to be applied to powders or mixtures of particles in the micrometre and nanometre range.

The reasons for including facilitating agents include, but are not limited to providing better dispersibility, control of agglomeration, the release or retention of the active particles from the delivery matrix. Examples of facilitating agents include, but are not limited to crosslinked PVP (crospovidone), cross linked carmellose (croscarmellose), sodium starch glycolate, Povidone (PVP), Povidone K12, Povidone K17, Povidone K25, Povidone K29/32 and Povidone K30, stearic acid, magnesium stearate, calcium stearate, sodium stearyl fumarate, sodium stearyl lactylate, zinc stearate, sodium stearate or lithium stearate, other solid state fatty acids such as oleic acid, lauric acid, palmitic acid, erucic acid, behenic acid, or derivatives (such as esters and salts), amnio acids such as leucine, isoleucine, lysine, valine, methionine, phenylalanine, aspartame or acesulfame K. The facilitating agent can be added to the milled mixture of biologically active material and co-grinding matrix and further processed in another milling device such as Mechnofusion, Cyclomixing, or impact milling such as ball milling, jet milling, or milling using a high pressure homogeniser, or combinations thereof. The facilitating agent can be added to the milling of the mixture of biologically active material and co-grinding matrix as some time before the end of the milling process.

Indomethacin can be milled with lactose monohydrate and alkyl sulfates. Indomethacin can be milled with lactose monohydrate and sodium lauryl sulfate. Indomethacin can be milled with lactose monohydrate and sodium octadecyl sulfate. Indomethacin can be milled with lactose monohydrate, alkyl sulfates and another surfactant or polymers. Indomethacin can be milled with lactose monohydrate, sodium lauryl sulfate and polyether sulfates. Indomethacin can be milled with lactose monohydrate, sodium lauryl sulfate and polyethylene glycol 40 stearate. Indomethacin can be milled with lactose monohydrate, sodium lauryl sulfate and polyethylene glycol 100 stearate. Indomethacin can be milled with lactose monohydrate, sodium lauryl sulfate and a poloxamer. Indomethacin can be milled with lactose monohydrate, sodium lauryl sulfate and poloxamer 407. Indomethacin can be milled with lactose monohydrate, sodium lauryl sulfate and poloxamer 338. Indomethacin can be milled with lactose monohydrate, sodium lauryl sulfate and poloxamer 188. Indomethacin can be milled with lactose monohydrate, sodium lauryl sulfate and a solid polyethylene glycol. Indomethacin can be milled with lactose monohydrate, sodium lauryl sulfate and polyethylene glycol 6000. Indomethacin can be milled with lactose monohydrate, sodium lauryl sulfate and polyethylene glycol 3000. Indomethacin can be milled with lactose monohydrate and polyether sulfates. Indomethacin can be milled with lactose monohydrate and polyethylene glycol 40 stearate. Indomethacin can be milled with lactose monohydrate and polyethylene glycol 100 stearate. Indomethacin can be milled with lactose monohydrate and polyvinyl-pyrrolidine. Indomethacin can be milled with lactose monohydrate and polyvinyl-pyrrolidone with an approximate molecular weight of 30,000-40,000. Indomethacin can be milled with lactose monohydrate and alkyl sulfonates. Indomethacin can be milled with lactose monohydrate and docusate sodium. Indomethacin can be milled with lactose monohydrate and a surfactant. Indomethacin can be milled with lactose monohydrate and lecithin. Indomethacin can be milled with lactose monohydrate and sodium n-lauroyl sarcosine. Indomethacin can be milled with lactose monohydrate and polyoxyethylene alkyl ether surfactants. Indomethacin can be milled with lactose monohydrate and PEG 6000. Indomethacin can be milled with lactose monohydrate and silica. Indomethacin can be milled with lactose monohydrate and Aerosil R972 fumed silica. Indomethacin can be milled with with lactose monohydrate, tartaric acid and sodium lauryl sulfate. In another red embodiment, indomethacin is milled with with lactose monohydrate, sodium bicarbonate and sodium lauryl sulfate. Indomethacin can be milled with lactose monohydrate, potassium bicarbonate and sodium lauryl sulfate.lndomethacin can be milled with mannitol and alkyl sulfates. Indomethacin can be milled with mannitol and sodium lauryl sulfate. Indomethacin can be milled with mannitol and sodium octadecyl sulfate. Indomethacin can be milled with mannitol, alkyl sulfates and another surfactant or polymers. Indomethacin can be milled with mannitol, sodium lauryl sulfate and polyether sulfates. Indomethacin can be milled with mannitol, sodium lauryl sulfate and polyethylene glycol 40 stearate. Indomethacin can be milled with mannitol, sodium lauryl sulfate and polyethylene glycol 100 stearate. Indomethacin can be milled with mannitol, sodium lauryl sulfate and a poloxamer. Indomethacin can be milled with mannitol, sodium lauryl sulfate and poloxamer 407. Indomethacin can be milled with mannitol, sodium lauryl sulfate and poloxamer 338. Indomethacin can be milled with mannitol, sodium lauryl sulfate and poloxamer 188. Indomethacin can be milled with mannitol, sodium lauryl sulfate and a solid polyethylene glycol. Indomethacin can be milled with mannitol, sodium lauryl sulfate and polyethylene glycol 6000. Indomethacin can be milled with mannitol, sodium lauryl sulfate and polyethylene glycol 3000. Indomethacin can be milled with mannitol and polyether sulfates. Indomethacin can be milled with mannitol and polyethylene glycol 40 stearate. Indomethacin can be milled with mannitol and polyethylene glycol 100 stearate Indomethacin can be milled with mannitol and polyvinyl-pyrrolidine. Indomethacin can be milled with mannitol and polyvinyl-pyrrolidone with an approximate molecular weight of 30,000-40,000. Indomethacin can be milled with mannitol and alkyl sulfonates. indomethacin can be milled with mannitol and docusate sodium. Indomethacin can be milled with mannitol and a surfactant. Indomethacin can be milled with mannitol and lecithin. Indomethacin can be milled with mannitol and sodium n-lauroyl sarcosine. Indomethacin can be milled with mannitol and polyoxyethylene alkyl ether surfactants. Indomethacin can be milled with mannitol and PEG 6000. Indomethacin can be milled with mannitol and silica. Indomethacin can be milled with mannitol and Aerosil R972 fumed silica. Indomethacin can be milled with with mannitol, tartaric acid and sodium lauryl sulfate. Indomethacin can be milled with with mannitol, sodium bicarbonate and sodium lauryl sulfate. Indomethacin can be milled with mannitol, potassium bicarbonate and sodium lauryl sulfate.

Naproxen can be milled with lactose monohydrate and alkyl sulfates. Naproxen can be milled with lactose monohydrate and sodium lauryl sulfate. Naproxen can be milled with lactose monohydrate and sodium octadecyl sulfate. Naproxen can be milled with lactose monohydrate, alkyl sulfates and another surfactant or polymers. Naproxen can be milled with lactose monohydrate, sodium lauryl sulfate and polyether sulfates. Naproxen can be milled with lactose monohydrate, sodium lauryl sulfate and polyethylene glycol 40 stearate. Naproxen can be milled with lactose monohydrate, sodium lauryl sulfate and polyethylene glycol 100 stearate. Naproxen can be milled with lactose monohydrate, sodium lauryl sulfate and a poloxamer. Naproxen can be milled with lactose monohydrate, sodium lauryl sulfate and poloxamer 407. Naproxen can be milled with lactose monohydrate, sodium lauryl sulfate and poloxamer 338. Naproxen can be milled with lactose monohydrate, sodium lauryl sulfate and poloxamer 188. Naproxen can be milled with lactose monohydrate, sodium lauryl sulfate and a solid polyethylene glycol. Naproxen can be milled with lactose monohydrate, sodium lauryl sulfate and polyethylene glycol 6000. Naproxen can be milled with lactose monohydrate, sodium lauryl sulfate and polyethylene glycol 3000. Naproxen can be milled with lactose monohydrate and polyether sulfates. Naproxen can be milled with lactose monohydrate and polyethylene glycol 40 stearate. Naproxen can be milled with lactose monohydrate and polyethylene glycol 100 stearate. Naproxen can be milled with lactose monohydrate and polyvinyl-pyrrolidine. Naproxen can be milled with lactose monohydrate and polyvinyl-pyrrolidone with an approximate molecular weight of 30,000-40,000. Naproxen can be milled with lactose monohydrate and alkyl sulfonates. Naproxen can be milled with lactose monohydrate and docusate sodium. Naproxen can be milled with lactose monohydrate and a surfactant. Naproxen can be milled with lactose monohydrate and lecithin. Naproxen can be milled with lactose monohydrate and sodium n-lauroyl sarcosine. Naproxen can be milled with lactose monohydrate and polyoxyethylene alkyl ether surfactants. Naproxen can be milled with lactose monohydrate and PEG 6000. Naproxen can be milled with lactose monohydrate and silica. Naproxen can be milled with lactose monohydrate and Aerosil R972 fumed silica. Naproxen can be milled with with lactose monohydrate, tartaric acid and sodium lauryl sulfate. Naproxen can be milled with with lactose monohydrate, sodium bicarbonate and sodium lauryl sulfate. Naproxen can be milled with lactose monohydrate, potassium bicarbonate and sodium lauryl sulfate. Naproxen can be milled with mannitol and alkyl sulfates. Naproxen can be milled with mannitol and sodium lauryl sulfate. Naproxen can be milled with mannitol and sodium octadecyl sulfate. Naproxen can be milled with mannitol, alkyl sulfates and another surfactant or polymers. Naproxen can be milled with mannitol, sodium lauryl sulfate and polyether sulfates. Naproxen can be milled with mannitol, sodium lauryl sulfate and polyethylene glycol 40 stearate. Naproxen can be milled with mannitol, sodium lauryl sulfate and polyethylene glycol 100 stearate. Naproxen can be milled with mannitol, sodium lauryl sulfate and a poloxamer. Naproxen can be milled with mannitol, sodium lauryl sulfate and poloxamer 407. Naproxen can be milled with mannitol, sodium lauryl sulfate and poloxamer 338. Naproxen can be milled with mannitol, sodium lauryl sulfate and poloxamer 188. Naproxen can be milled with mannitol, sodium lauryl sulfate and a solid polyethylene glycol. Naproxen can be milled with mannitol, sodium lauryl sulfate and polyethylene glycol 6000. Naproxen can be milled with mannitol, sodium lauryl sulfate and polyethylene glycol 3000. Naproxen can be milled with mannitol and polyether sulfates. Naproxen can be milled with mannitol and polyethylene glycol 40 stearate. Naproxen can be milled with mannitol and polyethylene glycol 100 stearate. Naproxen can be milled with mannitol and polyvinyl-pyrrolidine. Naproxen can be milled with mannitol and polyvinyl-pyrrolidone with an approximate molecular weight of 30,000-40,000. Naproxen can be milled with mannitol and alkyl sulfonates. Naproxen can be milled with mannitol and docusate sodium. Naproxen can be milled with mannitol and a surfactant. Naproxen can be milled with mannitol and lecithin. Naproxen can be milled with mannitol and sodium n-lauroyl sarcosine. Naproxen can be milled with mannitol and polyoxyethylene alkyl ether surfactants. Naproxen can be milled with mannitol and PEG 6000. Naproxen can be milled with mannitol and silica. Naproxen can be milled with mannitol and Aerosil R972 fumed silica. Naproxen can be milled with with mannitol, tartaric acid and sodium lauryl sulfate. Naproxen can be milled with with mannitol, sodium bicarbonate and sodium lauryl sulfate. Naproxen can be milled with mannitol, potassium bicarbonate and sodium lauryl sulfate.

Diclofenac can be milled with lactose monohydrate and alkyl sulfates. Diclofenac can be milled with lactose monohydrate and sodium lauryl sulfate. Diclofenac can be milled with lactose monohydrate and sodium octadecyl sulfate. Diclofenac can be milled with lactose monohydrate, alkyl sulfates and another surfactant or polymers. Diclofenac can be milled with lactose monohydrate, sodium lauryl sulfate and polyether sulfates. Diclofenac can be milled with lactose monohydrate, sodium lauryl sulfate and polyethylene glycol 40 stearate. Diclofenac can be milled with lactose monohydrate, sodium lauryl sulfate and polyethylene glycol 100 stearate. Diclofenac can be milled with lactose monohydrate, sodium lauryl sulfate and a poloxamer. Diclofenac can be milled with lactose monohydrate, sodium lauryl sulfate and poloxamer 407. Diclofenac can be milled with lactose monohydrate, sodium lauryl sulfate and poloxamer 338. Diclofenac can be milled with lactose monohydrate, sodium lauryl sulfate and poloxamer 188. Diclofenac can be milled with lactose monohydrate, sodium lauryl sulfate and a solid polyethylene glycol. Diclofenac can be milled with lactose monohydrate, sodium lauryl sulfate and polyethylene glycol 6000. Diclofenac can be milled with lactose monohydrate, sodium lauryl sulfate and polyethylene glycol 3000. Diclofenac can be milled with lactose monohydrate and polyether sulfates. Diclofenac can be milled with lactose monohydrate and polyethylene glycol 40 stearate. Diclofenac can be milled with lactose monohydrate and polyethylene glycol 100 stearate. Diclofenac can be milled with lactose monohydrate and polyvinyl-pyrrolidine. Diclofenac can be milled with lactose monohydrate and polyvinyl-pyrrolidone with an approximate molecular weight of 30,000-40,000. Diclofenac can be milled with lactose monohydrate and alkyl sulfonates. Diclofenac can be milled with lactose monohydrate and docusate sodium. Diclofenac can be milled with lactose monohydrate and a surfactant. Diclofenac can be milled with lactose monohydrate and lecithin. Diclofenac can be milled with lactose monohydrate and sodium n-lauroyl sarcosine. Diclofenac can be milled with lactose monohydrate and polyoxyethylene alkyl ether surfactants. Diclofenac can be milled with lactose monohydrate and PEG 6000. Diclofenac can be milled with lactose monohydrate and silica. Diclofenac can be milled with lactose monohydrate and Aerosil R972 fumed silica. Diclofenac can be milled with with lactose monohydrate, tartaric acid and sodium lauryl sulfate. Diclofenac can be milled with with lactose monohydrate, sodium bicarbonate and sodium lauryl sulfate. Diclofenac can be milled with lactose monohydrate, potassium bicarbonate and sodium lauryl sulfate. Diclofenac can be milled with mannitol and alkyl sulfates. Diclofenac can be milled with mannitol and sodium lauryl sulfate. Diclofenac can be milled with mannitol and sodium octadecyl sulfate. Diclofenac can be milled with mannitol, alkyl sulfates and another surfactant or polymers. Diclofenac can be milled with mannitol, sodium lauryl sulfate and polyether sulfates. Diclofenac can be milled with mannitol, sodium lauryl sulfate and polyethylene glycol 40 stearate. Diclofenac can be milled with mannitol, sodium lauryl sulfate and polyethylene glycol 100 stearate. Diclofenac can be milled with mannitol, sodium lauryl sulfate and a poloxamer. Diclofenac can be milled with mannitol, sodium lauryl sulfate and poloxamer 407. Diclofenac can be milled with mannitol, sodium lauryl sulfate and poloxamer 338. Diclofenac can be milled with mannitol, sodium lauryl sulfate and poloxamer 188. Diclofenac can be milled with mannitol, sodium lauryl sulfate and a solid polyethylene glycol. Diclofenac can be milled with mannitol, sodium lauryl sulfate and polyethylene glycol 6000. Diclofenac can be milled with mannitol, sodium lauryl sulfate and polyethylene glycol 3000. Diclofenac can be milled with mannitol and polyether sulfates. Diclofenac can be milled with mannitol and polyethylene glycol 40 stearate Diclofenac can be milled with mannitol and polyethylene glycol 100 stearate Diclofenac can be milled with mannitol and polyvinyl-pyrrolidine. Diclofenac can be milled with mannitol and polyvinyl-pyrrolidone with an approximate molecular weight of 30,000-40,000. Diclofenac can be milled with mannitol and alkyl sulfonates. Diclofenac can be milled with mannitol and docusate sodium. Diclofenac can be milled with mannitol and a surfactant. Diclofenac can be milled with mannitol and lecithin. Diclofenac can be milled with mannitol and sodium n-lauroyl sarcosine. Diclofenac can be milled with mannitol and polyoxyethylene alkyl ether surfactants. Diclofenac can be milled with mannitol and PEG 6000. Diclofenac can be milled with mannitol and silica. Diclofenac can be milled with mannitol and Aerosil R972 fumed silica. Diclofenac can be milled with with mannitol, tartaric acid and sodium lauryl sulfate. Diclofenac can be milled with with mannitol, sodium bicarbonate and sodium lauryl sulfate. Diclofenac can be milled with mannitol, potassium bicarbonate and sodium lauryl sulfate. Meloxicam can be milled with lactose monohydrate and alkyl sulfates. Meloxicam can be milled with lactose monohydrate and sodium lauryl sulfate. Meloxicam can be milled with lactose monohydrate and sodium octadecyl sulfate. Meloxicam can be milled with lactose monohydrate, alkyl sulfates and another surfactant or polymers. Meloxicam can be milled with lactose monohydrate, sodium lauryl sulfate and polyether sulfates. Meloxicam can be milled with lactose monohydrate, sodium lauryl sulfate and polyethylene glycol 40 stearate. Meloxicam can be milled with lactose monohydrate, sodium lauryl sulfate and polyethylene glycol 100 stearate. Meloxicam can be milled with lactose monohydrate, sodium lauryl sulfate and a poloxamer. Meloxicam can be milled with lactose monohydrate, sodium lauryl sulfate and poloxamer 407. Meloxicam can be milled with lactose monohydrate, sodium lauryl sulfate and poloxamer 338. Meloxicam can be milled with lactose monohydrate, sodium lauryl sulfate and poloxamer 188. Meloxicam can be milled with lactose monohydrate, sodium lauryl sulfate and a solidpolyethylene glycol. Meloxicam can be milled with lactose monohydrate, sodium lauryl sulfate and polyethylene glycol 6000. Meloxicam can be milled with lactose monohydrate, sodium lauryl sulfate and polyethylene glycol 3000. Meloxicam can be milled with lactose monohydrate and polyether sulfates. Meloxicam can be milled with lactose monohydrate and polyethylene glycol 40 stearate. Meloxicam can be milled with lactose monohydrate and polyethylene glycol 100 stearate. Meloxicam can be milled with lactose monohydrate and polyvinyl-pyrrolidine. Meloxicam can be milled with lactose monohydrate and polyvinyl-pyrrolidone with an approximate molecular weight of 30,000-40,000. Meloxicam is milled with lactose monohydrate and alkyl sulfonates. Meloxicam can be milled with lactose monohydrate and docusate sodium. Meloxicam is milled with lactose monohydrate and a surfactant. Meloxicam can be milled with lactose monohydrate and lecithin. Meloxicam can be milled with lactose monohydrate and sodium n-lauroyl sarcosine. Meloxicam can be milled with lactose monohydrate and polyoxyethylene alkyl ether surfactants. Meloxicam can be milled with lactose monohydrate and PEG 6000. Meloxicam can be milled with lactose monohydrate and silica. Meloxicam can be milled with lactose monohydrate and Aerosil R972 fumed silica. Meloxicam can be milled with with lactose monohydrate, tartaric acid and sodium lauryl sulfate. Meloxicam can be milled with with lactose monohydrate, sodium bicarbonate and sodium lauryl sulfate. Meloxicam can be milled with lactose monohydrate, potassium bicarbonate and sodium lauryl sulfate.Meloxicam can be milled with mannitol and alkyl sulfates. Meloxicam can be milled with mannitol and sodium lauryl sulfate. Meloxicam can be milled with mannitol and sodium octadecyl sulfate. Meloxicam is milled with mannitol, alkyl sulfates and another surfactant or polymers. Meloxicam can be milled with mannitol, sodium lauryl sulfate and polyether sulfates. Meloxicam can be milled with mannitol, sodium lauryl sulfate and polyethylene glycol 40 stearate. Meloxicam can be milled with mannitol, sodium lauryl sulfate and polyethylene glycol 100 stearate. Meloxicam can be milled with mannitol, sodium lauryl sulfate and a poloxamer. Meloxicam can be milled with mannitol, sodium lauryl sulfate and poloxamer 407. Meloxicam can be milled with mannitol, sodium lauryl sulfate and poloxamer 338. Meloxicam can be milled with mannitol, sodium lauryl sulfate and poloxamer 188. Meloxicam can be milled with mannitol, sodium lauryl sulfate and a solid polyethylene glycol. Meloxicam can be milled with mannitol, sodium lauryl sulfate and polyethylene glycol 6000. Meloxicam can be milled with mannitol, sodium lauryl sulfate and polyethylene glycol 3000. Meloxicam can be milled with mannitol and polyether sulfates. Meloxicam can be milled with mannitol and polyethylene glycol 40 stearate. Meloxicam can be milled with mannitol and polyethylene glycol 100 stearate Meloxicam can be milled with mannitol and polyvinyl-pyrrolidine. Meloxicam can be milled with mannitol and polyvinyl-pyrrolidone with an approximate molecular weight of 30,000-40,000. Meloxicam can be milled with mannitol and alkyl sulfonates. Meloxicam can be milled with mannitol and docusate sodium. Meloxicam can be milled with mannitol and a surfactant. Meloxicam can be milled with mannitol and lecithin. Meloxicam can be milled with mannitol and sodium n-lauroyl sarcosine. Meloxicam can be milled with mannitol and polyoxyethylene alkyl ether surfactants. Meloxicam can be milled with mannitol and PEG 6000. Meloxicam can be milled with mannitol and silica. Meloxicam can be milled with mannitol and Aerosil R972 fumed silica. Meloxicam can be milled with with mannitol, tartaric acid and sodium lauryl sulfate. Meloxicam can be milled with with mannitol, sodium bicarbonate and sodium lauryl sulfate. Meloxicam can be milled with mannitol, potassium bicarbonate and sodium lauryl sulfate.

Metaxalone can be milled with lactose monohydrate and alkyl sulfates. Metaxalone can be milled with lactose monohydrate and sodium lauryl sulfate. Metaxalone can be milled with lactose monohydrate and sodium octadecyl sulfate. Metaxalone can be milled with lactose monohydrate, alkyl sulfates and another surfactant or polymers. Metaxalone can be milled with lactose monohydrate, sodium lauryl sulfate and polyether sulfates. Metaxalone can be milled with lactose monohydrate, sodium lauryl sulfate and polyethylene glycol 40 stearate. Metaxalone can be milled with lactose monohydrate, sodium lauryl sulfate and polyethylene glycol 100 stearate. Metaxalone can be milled with lactose monohydrate, sodium lauryl sulfate and a poloxamer. Metaxalone can be milled with lactose monohydrate, sodium lauryl sulfate and poloxamer 407. Metaxalone can be milled with lactose monohydrate, sodium lauryl sulfate and poloxamer 338. Metaxalone can be milled with lactose monohydrate, sodium lauryl sulfate and poloxamer 188. Metaxalone can be milled with lactose monohydrate, sodium lauryl sulfate and a solid polyethylene glycol. Metaxalone can be milled with lactose monohydrate, sodium lauryl sulfate and polyethylene glycol 6000. Metaxalone can be milled with lactose monohydrate, sodium lauryl sulfate and polyethylene glycol 3000. Metaxalone can be milled with lactose monohydrate and polyether sulfates. Metaxalone can be milled with lactose monohydrate and polyethylene glycol 40 stearate. Metaxalone can be milled with lactose monohydrate and polyethylene glycol 100 stearate. Metaxalone can be milled with lactose monohydrate and polyvinyl-pyrrolidine. Metaxalone can be milled with lactose monohydrate and polyvinyl-pyrrolidone with an approximate molecular weight of 30,000-40,000. Metaxalone can be milled with lactose monohydrate and alkyl sulfonates. Metaxalone can be milled with lactose monohydrate and docusate sodium. Metaxalone can be milled with lactose monohydrate and a surfactant. Metaxalone can be milled with lactose monohydrate and lecithin. Metaxalone can be milled with lactose monohydrate and sodium n-lauroyl sarcosine. Metaxalone can be milled with lactose monohydrate and polyoxyethylene alkyl ether surfactants. Metaxalone can be milled with lactose monohydrate and PEG 6000. Metaxalone can be milled with lactose monohydrate and silica. Metaxalone can be milled with lactose monohydrate and Aerosil R972 fumed silica. Metaxalone can be milled with with lactose monohydrate, tartaric acid and sodium lauryl sulfate. Metaxalone can be milled with with lactose monohydrate, sodium bicarbonate and sodium lauryl sulfate. Metaxalone can be milled with with lactose monohydrate, sodium bicarbonate, poloxamer 407 and sodium lauryl sulfate. Metaxalone can be milled with lactose monohydrate, potassium bicarbonate and sodium lauryl sulfate. Metaxalone can be milled with with lactose monohydrate, potassium bicarbonate, poloxamer 407 and sodium lauryl sulfate. Metaxalone can be milled with mannitol and alkyl sulfates. Metaxalone can be milled with mannitol and sodium lauryl sulfate. Metaxalone can be milled with mannitol and sodium octadecyl sulfate. Metaxalone can be milled with mannitol, alkyl sulfates and another surfactant or polymers. Metaxalone can be milled with mannitol, sodium lauryl sulfate and polyether sulfates. Metaxalone can be milled with mannitol, sodium lauryl sulfate and polyethylene glycol 40 stearate. Metaxalone can be milled with mannitol, sodium lauryl sulfate and polyethylene glycol 100 stearate. Metaxalone can be milled with mannitol, sodium lauryl sulfate and a poloxamer. Metaxalone can be milled with mannitol, sodium lauryl sulfate and poloxamer 407. Metaxalone can be milled with mannitol, sodium lauryl sulfate and poloxamer 338. Metaxalone can be milled with mannitol, sodium lauryl sulfate and poloxamer 188. Metaxalone can be milled with mannitol, sodium lauryl sulfate and a solid polyethylene glycol. Metaxalone can be milled with mannitol, sodium lauryl sulfate and polyethylene glycol 6000. Metaxalone can be milled with mannitol, sodium lauryl sulfate and polyethylene glycol 3000. Metaxalone can be milled with mannitol and polyether sulfates. Metaxalone can be milled with mannitol and polyethylene glycol 40 stearate Metaxalone can be milled with mannitol and polyethylene glycol 100 stearate Metaxalone can be milled with mannitol and polyvinyl-pyrrolidine. Metaxalone can be milled with mannitol and polyvinyl-pyrrolidone with an approximate molecular weight of 30,000-40,000. Metaxalone can be milled with mannitol and alkyl sulfonates. Metaxalone can be milled with mannitol and docusate sodium. Metaxalone can be milled with mannitol and a surfactant. Metaxalone can be milled with mannitol and lecithin. Metaxalone can be milled with mannitol and sodium n-lauroyl sarcosine. Metaxalone can be milled with mannitol and polyoxyethylene alkyl ether surfactants. Metaxalone can be milled with mannitol and PEG 6000.Metaxalone can be milled with mannitol and silica. Metaxalone can be milled with mannitol and Aerosil R972 fumed silica. Metaxalone can be milled with with mannitol, tartaric acid and sodium lauryl sulfate. Metaxalone can be milled with with mannitol, sodium bicarbonate and sodium lauryl sulfate. Metaxalone can be milled with mannitol, potassium bicarbonate and sodium lauryl sulfate. Metaxalone can be milled with mannitol, sodium bicarbonate and sodium lauryl sulphate and Polxamer 407. Metaxalone can be milled with mannitol, potassium bicarbonate and sodium lauryl sulphate and Polxamer 407.

Disclosed is a biologically active material produced by the method described herein and composition comprising the biologically active material as described herein. The average particle size, determined on a particle number basis, can be equal to or less than a size selected from the group 10,000nm, 8000nm, 6000nm, 5000nm, 4000nm, 3000nm, 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The average particle size can be equal to or greater than 25nm. The particles can have a median particle size, determined on a particle volume basis, equal or less than a size selected from the group 20000nm, 15000nm, 10000 nm, 7500nm, 5000nm, 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The median particle size can be equal to or greater than 25nm. The percentage of particles, on a particle volume basis, can be selected from the group consisting of: 50%, 60%, 70%, 80%, 90%, 95% and 100% less than 2000nm (% < 2000 nm). The percentage of particles, on a particle volume basis, can be selected from the group consisting of: 50%, 60%, 70%, 80%, 90%, 95% and 100% less than 1000nm (% < 1000 nm). The percentage of particles, on a particle volume basis, can be selected from the group 0%, 10%, 20%, 30%, 40%, 50 %, 60%, 70%, 80%, 90%, 95% and 100 % less than 500nm (% < 500 nm). The percentage of particles, on a particle volume basis, can be selected from the group 0%, 10%, 20%, 30%, 40%, 50 %, 60%, 70%, 80%, 90%, 95% and 100 % less than 300nm (% < 300 nm). The percentage of particles, on a particle volume basis, can be selected from the group 0%, 10%, 20%, 30%, 40%, 50%, 60%, 70%, 80%, 90%, 95% and 100% less than 200nm (% < 200 nm). The Dx of the particle size distribution, as measured on a particle volume basis, can be selected from the group consisting of less than or equal to 10,000nm, 5000nm, 3000nm, 2000nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm, and 100 nm; wherein x is greater than or equal to 90. The biologically active material comprised in the composition can be selected from the group consisting of: fungicides, pesticides, herbicides, seed treatments, cosmeceuticals, cosmetics, complementary medicines, natural products, vitamins, nutrients, neutraceuticals, pharmaceutical actives, biologics, amino acids, proteins, peptides, nucleotides, nucleic acids, additives, foods and food ingredients and analogs, homologs and first order derivatives thereof. The biologically active material can be selected from the group consisting of: anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, cough suppressants (expectorants and mucolytics), diagnostic agents, diagnostic imaging agents, diuretics, dopaminergics (anti-parkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthenes. The biologically active material can be selected from the group consisting of: indomethacin, diclofenac, naproxen, meloxicam, metaxalone, cyclosporin A, progesterone celecoxib, cilostazol, ciprofloxacin, 2,4-dichlorophenoxyacetic acid, anthraquinone, creatine monohydrate, glyphosate, halusulfuron, mancozeb, metsulfuron, salbutamol, sulphur, tribenuran and estradiol or any salt or derivative thereof.

The biologically active material can be selected from the group consisting of: anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics. Cosmeceuticals, cosmetics, complementary medicines, natural products, vitamins, nutrients and nutraceuticals can be selected from the group consisting of: Glycolic acids, Lactic acids, , Carrageenan, Almonds, Mahogany wood, Andrographis Paniculata, Aniseed, Anthemis nobilis (chamomile), Apricot kernel, leaves of bearberry, leaves of cranberry, leaves of blueberry, leaves of pear trees, beta-carotene, black elderberry, black raspberry, black walnut shell, blackberry, bladderwrack, bletilla striata, borage seed, boysenberry, brazil nut, burdock root, butcher's broom extract , calamine, calcium gluconate, calendula, carnosic acid , Cantella asiatica, charcoal, chaste tree fruit , Chicory root extract, chitosan, choline, cichorium intybus, clematis vitalba, coffea Arabica, coumarin, crithmum maritimum, curcumin, coffee, cocoa, cocoa powder, cocoa nibs, cocoa mass, cocoa liquor, cocoa products, dogwood, Echinacea, echium lycopsis, anise, atragalus, bilberry, bitter orange, black cohosh, cat's claw, chamomile, chasteberry, cranberry, dandelion, Echinacea, ephedra, European elder Epilobium angustifolium, horse chestnut, cloves, evening primrose, fennel seed, fenugreek, feverfew, flaxseed, fumaria officinalis, garlic, geranium, ginger, ginkgo, ginseng, goldenseal, grape seed, green tea, guava, hawthorn, hayflower, hazelnut, helichrysum, hoodia, horseradish, mulbe italicum, hibiscus, hierochloe odorata, hops, horse chestnut, ilex paraguariensis, indian gooseberry, irish moss, juniper berry, kudzu root, lady's thistle, lavender, lemongrass, lentius edodes, licorice, longifolene, loquat, lotus seed, luffa cylindrica, lupine, maroinberry, marjoram, meadowsweet, milk vetch root, mimosa tenuiflora, mistletoe, mulberry, noni, kelp, oatmeal, oregano, papaya, parsley, peony root, pomegranate, pongamia glabra seed, pongamia pinnata, quinoa seed, red raspberry, rose hip, rosemary, sage, saw palmetto, soy bean, szechuan peppercorn, tephrosia purpurea, terminalia catappa, terminalia sericea, thunder god vine, thyme, turmeric, valeriana officinalis, walnuts, white tea leaf, yam, witch hazel, wormwood, yarrow, valerian, yohimbe, mangosteen, sour sob, goji berry, spirulina and durian skin.

Disclosed are compositions comprising the biologically active ingredient together with a grinding matrix, a mixture of grinding matrix materials, milling aids, mixtures of milling aids, facilitating agents and/or mixtures of facilitating agents as described herein, in concentrations and ratios as described herein under the methods disclosed herein.

Disclosed is a pharmaceutical composition comprising a biologically active material produced by the method described herein and compositions described herein. Disclosed are pharmaceutical compositions comprising the biologically active ingredient together with a grinding matrix, a mixture of grinding matrix materials, milling aids, mixtures of milling aids, facilitating agents and/or mixtures of facilitating agents as described herein, in concentrations and ratios as described herein under the methods disclosed herein. The average particle size, determined on a particle number basis, can be equal to or less than a size selected from the group 10,000nm, 8000nm, 6000nm, 5000nm, 4000nm, 3000nm, 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The average particle size can be equal to or greater than 25nm. The particles can have a median particle size, determined on a particle volume basis, equal or less than a size selected from the group 20000nm, 15000nm, 10000 nm, 7500nm, 5000nm, 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The median particle size can be equal to or greater than 25nm. The percentage of particles, on a particle volume basis, can be selected from the group consisting of: 50%, 60%, 70%, 80%, 90%, 95% and 100 % less than 2000nm (% < 2000 nm). The percentage of particles, on a particle volume basis, can be selected from the group consisting of: 50%, 60%, 70%, 80%, 90%, 95% and 100 % less than 1000nm (% < 1000 nm). The percentage of particles, on a particle volume basis, can be selected from the group 0%, 10%, 20%, 30%, 40%, 50%, 60%, 70%, 80%, 90%, 95% and 100 % less than 500nm (% < 500 nm). The percentage of particles, on a particle volume basis, can be selected from the group 0%, 10%, 20%, 30%, 40%, 50%, 60%, 70%, 80%, 90%, 95% and 100 % less than 300nm (% < 300 nm). The percentage of particles, on a particle volume basis, can be selected from the group 0%, 10%, 20%, 30%, 40%, 50%, 60%, 70%, 80%, 90%, 95% and 100 % less than 200nm (% < 200 nm).

The crystallinity profile of the biologically active material can be selected from the group consisting of: at least 50% of the biologically active material is crystalline, at least 60% of the biologically active material is crystalline, at least 70% of the biologically active material is crystalline, at least 75% of the biologically active material is crystalline, at least 85% of the biologically active material is crystalline, at least 90% of the biologically active material is crystalline, at least 95% of the biologically active material is crystalline and at least 98% of the biologically active material is crystalline. The crystallinity profile of the biologically active material can be substantially equal to the crystallinity profile of the biologically active material before the material was subject to the method described herein. The amorphous content of the biological active material can be selected from the group consisting of: less than 50% of the biologically active material is amorphous, less than 40% of the biologically active material is amorphous, less than 30% of the biologically active material is amorphous, less than 25% of the biologically active material is amorphous, less than 15% of the biologically active material is amorphous, less than 10% of the biologically active material is amorphous, less than 5% of the biologically active material is amorphous and less than 2% of the biologically active material is amorphous. The biologically active material might have had no significant increase in amorphous content following subjecting the material to the method as described herein.

The biologically active material can be selected from the group consisting of: fungicides, pesticides, herbicides, seed treatments, cosmeceuticals, cosmetics, complementary medicines, natural products, vitamins, nutrients, neutraceuticals, pharmaceutical actives, biologics, amino acids, proteins, peptides, nucleotides, nucleic acids, additives, foods and food ingredients and analogs, homologs and first order derivatives thereof. Where the biologically active material is a naturally occurring material or a derivate of a naturally occuring material, such as but not limited to, seeds, cocoa and cocoa solids, coffee, herbs, spices, other plant materials, minerals, animal products, shells and other skeletal material, the particles can have a median particle size, determined on a particle volume basis, equal or less than a size selected from the group 10,000nm, 8000nm, 6000nm, 5000nm, 4000nm and 3000nm. The biologically active material can be selected from the group consisting of: anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, cough suppressants (expectorants and mucolytics), diagnostic agents, diagnostic imaging agents, diuretics, dopaminergics (anti-parkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthenes. The biologically active material can be selected from the group consisting of: indomethacin, diclofenac, naproxen, meloxicam, metaxalone, cyclosporin A, progesterone celecoxib, cilostazol, ciprofloxacin, 2,4-dichlorophenoxyacetic acid, anthraquinone, creatine monohydrate, glyphosate, halusulfuron, mancozeb, metsulfuron, salbutamol, sulphur, tribenuran and estradiol or any salt or derivative thereof.

Disclosed is a method of treating a human in need of such treatment comprising the step of administering to the human an effective amount of a pharmaceutical composition as described herein.

Disclosed is the use of a pharmaceutical composition as described herein in the manufacture of a medicament for the treatment of a human in need of such treatment.

Diclosed is a method for manufacturing a pharmaceutical composition as described herein comprising the step of combining a therapeutically effective amount of a biologically active material prepared by a method described herein or a composition as described herein, together with a pharmaceutically acceptable carrier to produce a pharmaceutically acceptable dosage form.

Disclosed is a method for manufacturing a veterinary product comprising the step of combining a therapeutically effective amount of the biologically active material prepared by a method as described herein or a composition as described herein, together with an acceptable excipient to produce a dosage form acceptable for veterinary use.

Disclosed is a method for manufacturing an agricultural product comprising the step of combining an effective amount of the biologically active material prepared by a method described herein or a composition as described herein. The agricultural product can be combined with an acceptable excipient to produce a formulation such as, but not limited to a water dispersible granule, wettable granule, dry flowable granule or soluble granule that is used to prepare a solution for use in agricultural applications. The product can be selected from the group consisting of: herbicides, pesticides, seed treatments, herbicide safeners, plant growth regulators and fungicides. The methods disclosed herein can be used to increase the dissolution of the biologically active material particles in water or other solvents, resulting in better, faster or more complete preparation and mixing. This will result in a more consistent product performance such as better weed, disease and pest control and other practical benefits such as faster machinery, tank and sprayer cleanout, less rinsate, and a reduced impact on the environment. Disclosed is a method to produce powders that have active particles with a high surface area. Such powders would provide better performance in areas such as seed treatment where dry powders are applied to seeds as fungicides, herbicide safeners, plant growth regulators and other treatments. The higher surface area would provide more activity per mass of active used. Actives such as pesticides, fungicides and seed treatments subject to the method of can be formulated to produce suspensions of the actives when added to water or other solvents. As these suspensions will have particles of very small size and high surface area they will possess at least three highly desirable traits. The first is that small particles with high surface area will adhere better to surfaces such as leafs and other foliage that the suspension is applied to. This will result in better rain fastness and a longer period of activity. The second aspect is that smaller particles with a higher surface area deliver superior coverage per unit mass of active applied. For example, if 100 particles are needed on a leaf and if the particle diameter is reduced to one third of the former diameter by the methods disclosed herein, then the dosage can be reduced to about 11 % of the former dosage, resulting in lower cost, less residue on harvested crops, and mitigation of environmental impact. In another embodiment the smaller particles will deliver better bioavailability. With many low solubility actives, such as fungicides and pesticides the particles that adhere to plant material slowly dissolve over days and weeks providing continued protection from disease and pests. With this method disclosed herein beingable to deliver better bioavailability in many circumstances it will be possible to reduce the amount of active that needs to be applied. As with the second aspect such an outcome would lower costs, minimize residues and mitigate environmental impact. The powder produced in the milling process can be be subject to a process such as wet or dry granulation that makes the powder free flowing and low in dust content yet easily dispersible once in water or other solvent.

The biologically active material can be a herbicide, pesticide, seed treatment, herbicide safener, plant growth regulator or fungicide selected from the group consisting of: 2-phenylphenol, 8-hydroxyquinoline sulfate, acibenzolar, allyl alcohol, azoxystrobin, basic benomyl, benzalkonium chloride, biphenyl, blasticidin-S, Bordeaux mixture, Boscalid, Burgundy mixture, butylamine, Cadendazim, calcium polysulfide, Captan, carbamate fungicides, carbendazim, carvone, chloropicrin, chlorothalonil, ciclopirox, clotrimazole, conazole fungicides, Copper hydroxide, copper oxychloride, copper sulfate, copper(II) carbonate, copper(II) sulfate, cresol, cryprodinil, cuprous oxide, cycloheximide, Cymoxanil, DBCP, dehydroacetic acid, dicarboximide fungicides, difenoconazole, dimethomorph, diphenylamine, disulfiram, ethoxyquin, famoxadone, fenamidone, Fludioxonil, formaldehyde, fosetyl, Fosetyl-aluminium, furfural, griseofulvin, hexachlorobenzene, hexachlorobutadiene, hexachlorophene, hexaconazole, imazalil, Imidacloprid, iodomethane, Iprodione, Lime sulfur, mancozeb, mercuric chloride, mercuric oxide, mercurous chloride, Metalaxyl, metam, methyl bromide, methyl isothiocyanate, metiram, natamycin, nystatin, organotin fungicides, oxythioquinox, pencycuron, pentachlorophenol, phenylmercury acetate, potassium thiocyanate, procymidone, propiconazole, propineb, pyraclostrobin, pyrazole fungicides, pyridine fungicides, pyrimethanil, pyrimidine fungicides, pyrrole fungicides, quinoline fungicides, quinone fungicides, sodium azide, streptomycin, sulfur, Tebucanazole, thiabendazole, thiomersal, tolnaftate, Tolylfluanid, triadimersol, tributyltin oxide, Trifloxystrobin, triflumuron, Undecylenic acid, urea fungicides, vinclozolin, Ziram,3-dihydro-3-methyl-1, 3-thiazol-2-ylidene-xylidene, 4-D esters, 4-DB esters, 4-parathion methyl, Acetamiprid, aclonifen, acrinathrin, alachlor, allethrin, alpha-cypermethrin, Aluminium phosphide, amitraz, anilophos, azaconazole, azinphos-ethyl, azinphos-methyl, benalaxyl, benfluralin, benfuracarb, benfuresate, bensulide, benzoximate, benzoylprop-ethyl, betacyfluthrin, beta-cypermethrin, bifenox, bifenthrin, binapacryl, bioallethrin, bioallethrin S, bioresmethrin, biteranol, Brodifacoum, bromophos, bromopropylate, bromoxynil, bromoxynil esters, bupirimate, buprofezin, butacarboxim, butachlor, butamifos, butoxycarboxin, butralin, butylate, calcium sulfate, cambda-cyhalothrin, carbetamide, carboxin, chlordimeform, chlorfenvinphos, chlorflurazuron, chlormephos, chlornitrofen, chlorobenzilate, chlorophoxim, chloropropylate, chlorpropham, Chlorpyrifos, chlorpyrifos-methyl, cinmethylin, clethodim, clomazone, clopyralid esters, CMPP esters, cyanophos, cycloate, cycloprothrin, cycloxydim, cyfluthrin, cyhalothrin, cypermethrin, cyphenothrin, cyproconazole, deltamethrin, demeton-S-methyl, desmedipham, dichlorprop esters, dichlorvos, diclofop-methyldiethatyl, dicofol, 1, difenoconazole, dimethachlor, dimethomoph, diniconazole, dinitramine, dinobuton, dioxabenzafos, dioxacarb, disulfoton, ditalimfos, dodemorph, dodine, edifenphos, emamectin, empenthrin, endosulfan, EPNethiofencarb, epoxyconazole, esfenvalerate, ethalfluralin, ethofumesate, ethoprophos, ethoxyethyl, etofenprox, etridiazole, etrimphos, Famoxadone, fenamiphos, fenarimol, fenazaquin, fenitrothion, fenobucarb, fenoxapropethyl, fenoxycarb, fenpropathrin, fenpropidin, fenpropimorph, fenthiocarb, fenthion, fenvalerate, fluazifop, fluazifop-P, fluchloralin, flucythrinate, flufenoxim, flufenoxuron, flumetralin, fluorodifen, fluoroglycofen ethyl, fluoroxypyr esters, flurecol butyl, flurochloralin, flusilazole, formothion, gamma-HCH, haloxyfop, haloxyfop-methyl, hexaflumuron, hydroprene, imibenconazole, indoxacarb, ioxynil esters, isofenphos, isoprocarb, isopropalin, isoxathion, malathion, maneb, MCPA esters, mecoprop-P esters, mephospholan, Metaldehyde, methidathion, Methomyl, methoprene, methoxychlor, metolachlor, mevinphos, monalide, myclobutanil, N-2, napropamide, nitrofen, nuarimol, oxadiazon, oxycarboxin, oxyfluorfen, penconazole, pendimethalin, permethrin, phenisopham, phenmedipham, phenothrin, phenthoate, phosalone, phosfolan, phosmet, picloram esters, pirimicarb, pirimiphos-ethyl, pirimiphos-methyl, pretilachlor, prochloraz, profenofos, profluralin, promecarb, propachlor, propanil, propaphos, propaquizafop, propargite, propetamphos, pymetrozine, pyrachlofos, pyridate, pyrifenox, quinalphos, quizalofop-P, resmethrin, Spinetoram J, Spinetoram L, Spinosad A, Spinosad B, tau-fluvalinate, tebuconazole, Tebufenozide, tefluthrin, temephos, terbufos, tetrachlorinphos, tetraconazole, tetradifon, tetramethrin, Thiamethoxam, tolclofos-methyl, tralomethrin, triadimefon, triadimenol, triazophos, triclopyr esters, tridemorph, tridiphane, triflumizole, trifluralin, xylylcarb, 3-dihydro-3-methyl-1, 3-thiazol-2-ylidene-xylidene, 4-D esters, 4-DB esters, 4-parathion methyl, Acetamiprid, acetochlor, aclonifen, acrinathrin, alachlor, allethrin, alpha-cypermethrin, Aluminium phosphide, amitraz, anilophos, azaconazole, azinphos-ethyl, azinphos-methyl, benalaxyl, benfluralin, benfuracarb, benfuresate, bensulide, benzoximate, benzoylprop-ethyl, betacyfluthrin, beta-cypermethrin, bifenox, bifenthrin, binapacryl, bioallethrin, bioallethrin S, bioresmethrin, biteranol, Brodifacoum, bromophos, bromopropylate, bromoxynil, bromoxynil esters, bupirimate, buprofezin, Butacarboxim, butachlor, butamifos, butoxycarboxin, butralin, butylate, calcium sulfate, cambda-cyhalothrin, carbetamide, carboxin, chlordimeform, chlorfenvinphos, chlorflurazuron, chlormephos, chlornitrofen, chlorobenzilate, chlorophoxim, chloropropylate, chlorpropham, Chlorpyrifos, chlorpyrifos-methyl, cinmethylin, clethodim, clomazone, clopyralid esters, CMPP esters, cyanophos, cycloate, cycloprothrin, cycloxydim, cyfluthrin, cyhalothrin, cypermethrin, cyphenothrin, cyproconazole, deltamethrin, demeton-S-methyl, desmedipham, dichlorprop esters, dichlorvos, diclofop-methyldiethatyl, dicofol, dimethachlor, dimethomoph, diniconazole, dinitramine, dinobuton, dioxabenzafos, dioxacarb, disulfoton, ditalimfos, dodemorph, dodine, edifenphos, emamectin, empenthrin, endosulfan, EPNethiofencarb, epoxyconazole, esfenvalerate, ethalfluralin, ethofumesate, ethoprophos, ethoxyethyl, ethoxyquin, etofenprox, etridiazole, etrimphos, fenamiphos, fenarimol, fenazaquin, fenitrothion, fenobucarb, fenoxapropethyl, fenoxycarb, fenpropathrin, fenpropidin, fenpropimorph, fenthiocarb, fenthion, fenvalerate, fluazifop, fluazifop-P, fluchloralin, flucythrinate, flufenoxim, flufenoxuron, flumetralin, fluorodifen, fluoroglycofen ethyl, fluoroxypyr esters, flurecol butyl, flurochloralin, flusilazole, formothion, gamma-HCH, haloxyfop, haloxyfop-methyl, hexaflumuron, hydroprene, imibenconazole, indoxacarb, ioxynil esters, isofenphos, isoprocarb, isopropalin, isoxathion, malathion, maneb, MCPA esters, mecoprop-P esters, mephospholan, Metaldehyde, methidathion, Methomyl, methoprene, methoxychlor, mevinphos, monalide, myclobutanil, myclobutanil, N-2, napropamide, nitrofen, nuarimol, oxadiazon, oxycarboxin, oxyfluorfen, penconazole, permethrin, phenisopham, phenmedipham, phenothrin, phenthoate, phosalone, phosfolan, phosmet, picloram esters, pirimicarb, pirimiphos-ethyl, pirimiphos-methyl, pretilachlor, prochloraz, profenofos, profluralin, promecarb, propachlor, propanil, propaphos, propaquizafop, propargite, propetamphos, pymetrozine, pyridate, pyrifenox, quinalphos, quizalofop-P, resmethrin, Spinetoram J, Spinetoram L, Spinosad A, Spinosad B, tau-fluvalinate, Tebufenozide, tefluthrin, temephos, terbufos, tetrachlorinphos, tetraconazole, tetradifon, tetramethrin, Thiamethoxam, tolclofos-methyl, tralomethrin, triadimenol, triazophos, triclopyr esters, tridemorph, tridiphane, triflumizole, trifluralin, xylylcarb and any combination thereof. Disclosed is a method for manufacturing of a pharmaceutical formulation comprising the step of combining an effective amount of the biologically active material prepared by a method described herein together with an acceptable excipients to produce a formulation that can deliver a therapeutically effect amount of active to the pulmonary or nasal area. Such a formulation could be, but is not limited to a dry powder formulation for oral inhalation to the lungs or a formulation for nasal inhalation. The method for manufacturing such a formulation can use lactose, mannitol, surcose, sorbitol, xylitol or other sugar or polyol as the co-grinding matrix together with surfactant such as, but not limited to lecithin, DPPC (dipalmitoyl phosphatidylcholine), PG (phosphatidylglycerol), dipalmitoyl phosphatidyl than olamine (DPPE), dipalmitoyl phosphatidylinositol (DPPI) or other phospholipid. The particle size of the material produced by the method disclosed herein results in the materials being readily aerosolized and suitable for methods of delivery to a subject in need thereof, including pulmonary and nasal delivery methods. Disclosed is a method for the manufacture of a composition for industrical application, such as, but not limited to paints, polymers or other functional coatings, comprising the step of combining an effective amount of the active material prepared by a method described herein together with an acceptable excipient to produce a composition that can deliver an active particle such as, but not limited to, a fungicide in solid form to a coating resistant to attack by biological agents such as, but not limited to, a fungus or algae. Because small particles provide a greater surface coverage of active agent per unit mass than conventionally sized particles less active is required in the composition. The particles generated by the method would also provide ascetic advantages as they can be incorporated into a coating formulation without the appearance of having particulate matter in the coating. The method for manufacturing such a composition can use titanium dioxide, silica, sodium chloride or other inorganic salts with a suitable surfactant or polymer. The active can be a fungicide selected from the list of herbicides, pesticides, seed treatments, herbicide safeners, plant growth regulators and fungicides described above.

Disclosed is a method for the manufacture of a radio-contrast agent for use in radiological examinations. A common example of such an agent would be barium sulfate which is commonly used in examinations of the gastrointestinal tract. Agents such as barium sulfate are essentially insoluble in water and function as discrete particles dispersed throughout the area of examination. Formulations of active material used as radio-contrast agents as prepared by a method described herein with other acceptable excipients could be used to provide enhanced sensitivity and lower toxicity due to the increased surface area provided by the particle size reduction. The increased surface area will provide greater coverage of the tissue to be measured providing better contrast. If the agent has toxic side effects greater contrast per unit mass would allow for less contrast agent to be used compared with conventional formulations. Another advantage of preparing such a formulation using the method described herein is the ability to administer that contrast agent as a dry formulation thus eliminating undesirable aspects of drinking a liquid formulation.

Disclosed is a method for the manufacture of a composition for use as a food product where the production of small particles has other functional advantages in addition to a faster dissolution of the active. One example would be where the active agent is cocoa or cocoa derived solids. When cocoa is processed in the manufacture of chocolate the particle size must be reduced below a size threshold such that the chocolate has a smooth feel when eaten. In the same way better flavour is thought to come from small cocoa particles. Premium chocolate is known to have a small particle size distribution. By combining an appropriate amount of the active material, such as cocoa, cocoa powder, cocoa nibs, cocoa mass or cocoa liquor prepared by a method described herein together with other food ingredients a food product such as chocolate can be prepared. This can be done to both enhance existing food products such as chocolate or provide a more efficient and less costly process for some aspects of the food product manufacture. Disclosed is the preparation of a food product for drinking by combining an appropriate amount of the active material, such as cocoa cocoa powder, cocoa nibs, cocoa mass, cocoa liquor or coffee, prepared by the method described herein together with other food ingredients. Materials produced using this method disclosed herein, having very small particles, could be directly used in drink products without leaving residue in the products due to large particle size. An example of this would be a drinking cocoa or drinking chocolate were a cocoa material could be milled with a matrix such as but not limited to sugar, glucose or lactose. Apart from greater release of flavours, such a product could directly use the natural product where conventional food products only use water soluble extracts. A clear example of this is coffee products. Instant coffee provides a convenient form of the product but is made by extracting flavor from coffee beans and then processing it into a soluble powder. In doing so some of the complex flavor of coffee is lost. In comparison, coffee made from ground coffee beans provides an enhanced flavor rich drink but requires greater preparation and often uses expensive apparatuses. Some coffee styles used ground coffee beans directly in a cup but this method leaves a thick sludge in the bottom of the cup. Material produced by the method described herein would overcome these limitations of the prior art. By preparing the composition from coffee beans the full flavor can be accessed and the small particle size produced by this method disclosed herein produces a drink where the particles are suspended in the liquid which do not form a thick sludge. A further advantage is that the material produced is a dry powder which can then be easily packaged or processed further to provide a saleable product.. A further advantage is that natural products such as coffee are encapsulated into the carrier matrix and thus have superior powder handling properties compared to natural products milled on there own. Materials such as coffee can be milled in high energy mills to produce particles with small size but the material is sticky and hard to handle. Other technologies, such as wet milling would be more costly as further processing, like spray drying, would be required to produce a powder. Matrices used for milling in this aspect can include, but are not limited to, lactose, sucrose, fructose, mannitol, glucose, xylitol, milk powders, other milk solids and lethicin. The particles can be a size equal to or less than 20,000nm. The particles can be a size equal to or less than 10,000nm.

The method enables production of highly water-soluble biologically active materials. Such materials may exhibit advantages over conventional materials by way of, for example, more rapid therapeutic action or lower dose. In contrast, wet grinding techniques utilizing water (or other comparably polar solvents) are incapable of being applied to such materials, as the particles dissolve appreciably in the solvent.

Other aspects and advantages will become apparent to those skilled in the art from a review of the ensuing description.

### Brief Description of the Drawings

Figure 1A. Powder charge composition and particle size distribution of material milled at volume percentages 25% or higher, examples A to S.
Figure 1B. Powder charge composition and particle size distribution of material milled at volume percentages 25% or higher, examples T to AL.
Figure 1C. Powder charge composition and particle size distribution of material milled at volume percentages 25% or higher, examples AM to AQ.
Figure 2A. Powder charge composition and particle size distribution of Naproxen Acid milled in Mannitol in a ½ Gallon 1S Attritor mill, examples A to M.
Figure 3A. Powder charge composition and particle size distribution of Naproxen Acid milled in SPEX mill and particle size distribution after filtration, examples A to L.

### Detailed Description

### General

Those skilled in the art will appreciate that the invention described herein is susceptible to variations and modifications other than those specifically described. It is to be understood that the invention includes all such variations and modifications. The invention also includes all of the steps, features, compositions and materials referred to or indicated in the specification, individually or collectively and any and all combinations or any two or more of the steps or features.
The invention described herein may include one or more ranges of values (e.g. size, concentration etc). A range of values will be understood to include all values within the range, including the values defining the range, and values adjacent to the range that lead to the same or substantially the same outcome as the values immediately adjacent to that value which defines the boundary to the range.

Throughout this specification, unless the context requires otherwise, the word "comprise" or variations, such as "comprises" or "comprising" will be understood to imply the inclusion of a stated integer, or group of integers, but not the exclusion of any other integers or group of integers. It is also noted that in this disclosure, and particularly in the claims and/or paragraphs, terms such as "comprises", "comprised", "comprising" and the like can have the meaning attributed to it in US Patent law; e.g., they can mean "includes", "included", "including", and the like.
"Therapeutically effective amount" as used herein with respect to methods of treatment and in particular drug dosage, shall mean that dosage that provides the specific pharmacological response for which the drug is administered in a significant number of subjects in need of such treatment. It is emphasized that "therapeutically effective amount," administered to a particular subject in a particular instance will not always be effective in treating the diseases described herein, even though such dosage is deemed a "therapeutically effective amount" by those skilled in the art. It is to be further understood that drug dosages are, in particular instances, measured as oral dosages, or with reference to drug levels as measured in blood.
The term "inhibit" is defined to include its generally accepted meaning which includes prohibiting, preventing, restraining, and lowering, stopping, or reversing progression or severity, and such action on a resultant symptom. Disclosed are both medical therapeutic and prophylactic administration, as appropriate.
The term "biologically active material" is defined to mean a biologically active compound or a substance which comprises a biologically active compound. In this definition, a compound is generally taken to mean a distinct chemical entity where a chemical formula or formulas can be used to describe the substance. Such compounds would generally, but not necessarily be identified in the literature by a unique classification system such as a CAS number. Some compounds may have a more complex and have a mixed chemical structure. For such compounds they may only have an empirical formula or be qualitatively indentified. A compound would generally be a pure material, although it would be expected that up to 10%, 20%, 30%, 40%, 50%, 60%, 70%, 80%, 90% of the substance could be other impurities and the like. Examples of biologically active compounds are, but not limited to, fungicides, pesticides, herbicides, seed treatments, cosmeceuticals, cosmetics, complementary medicines, natural products, vitamins, nutrients, neutraceuticals, pharmaceutical actives, biologics, amino acids, proteins, peptides, nucleotides, nucleic acids, additives, foods and food ingredients and analogs, homologs and first order derivatives thereof. A substance that contains a biological active compound is any substance which has as one of its components a biological active compound. Examples of substances containing biologically active compounds are, but not limited to, pharmaceutical formulations and products, cosmetic formulations and products, industrial formulations and products, agricultural formulations and products, foods, seeds, cocoa and cocoa solids, coffee, herbs, spices, other plant materials, minerals, animal products, shells and other skeletal material.
Any of the terms, "biological(ly) active", "active", "active material" shall have the same meaning as biologically active material.
The term "grinding matrix" is defined as any inert substance that a biologically active material can or is combined with and milled. The terms "co-grinding matrix" and "matrix" are interchangeable with "grinding matrix".

### Particle Size

There are a wide range of techniques that can be utilized to characterize the particle size of a material. Those skilled in the art also understand that almost all these techniques do not physically measure the actually particle size, as one might measure something with a ruler, but measure a physical phenomena which is interpreted to indicate a particle size. As part of the interpretation process some assumptions need to be made to enable mathematical calculations to be made. These assumptions deliver results such as an equivalent spherical particle size, or a hydrodynamic radius.

Amongst these various methods, two types of measurements are most commonly used. Photon correlation spectroscopy (PCS), also known as 'dynamic light scattering' (DLS) is commonly used to measure particles with a size less than 10 micron. Typically this measurement yields an equivalent hydrodynamic radius often expressed as the average size of a number distribution. The other common particle size measurement is laser diffraction which is commonly used to measure particle size from 100 nm to 2000 micron. This technique calculates a volume distribution of equivalent spherical particles that can be expressed using descriptors such as the median particle size or the % of particles under a given size.

Those skilled in the art recognize that different characterization techniques such as photon correlation spectroscopy and laser diffraction measure different properties of a particle ensemble. As a result multiple techniques will give multiple answers to the question, "what is the particle size." In theory one could convert and compare the various parameters each technique measures, however, for real world particle systems this is not practical. As a result the particle size will be given as two different sets of values that each relate to these two common measurement techniques, such that measurements could be made with either technique and then evaluated against the description provided herein.

For measurements made using a photo correlation spectroscopy instrument, or an equivalent method known in the art, the term "number average particle size" is defined as the average particle diameter as determined on a number basis.

For measurements made using a laser diffraction instrument, or an equivalent method known in the art, the term "median particle size" is defined as the median particle diameter as determined on an equivalent spherical particle volume basis. Where the term median is used, it is understood to describe the particle size that divides the population in half such that 50 % of the population is greater than or less than this size. The median particle size is often written as D50, D(0.50) or D[0.5] or similar. As used herein D50, D(0.50) or D[0.5] or similar shall be taken to mean 'median particle size'.

The term "Dx of the particle size distribution" refers to the xth percentile of the distribution; thus, D90 refers to the 90^{th} percentile, D95 refers to the 95^{th} percentile, and so forth. Taking D90 as an example this can often be written as, D(0.90) or D[0.9] or simialr. With respect to the median particle size and Dx an upper case D or lowercase d are interchangeable and have the same meaning.Another commonly used way of describing a particle size distribution measured by laser diffraction, or an equivalent method known in the art, is to describe what % of a distribution is under or over a nominated size. The term "percentage less than" also written as "%<" is defined as the percentage, by volume, of a particle size distribution under a nominated size -for example the % < 1000 nm. The term "percentage greater than" also written as "%>" is defined as the percentage, by volume, of a particle size distribution over a nominated size -for example the % > 1000 nm.

The particle size should be taken to mean the particle size as measured at or shortly before the time of use. For example, the particle size is measured 2 months after the material is subject to the milling method disclosed herein. The particle size can be measured at a time selected from the group consisting of: 1 day after milling, 2 days after milling, 5 days after milling, 1 month after milling, 2 months after milling, 3 months after milling, 4 months after milling, 5 months after milling, 6 months after milling, 1 year after milling, 2 years after milling, 5 years after milling.

For many of the materials subject to the methods disclosed herein the particle size can be easily measured. Where the active material has poor water solubility and the matrix it is milled in has good water solubility the powder can simply be dispersed in an aqueous solvent. In this scenario the matrix dissolves leaving the active material dispersed in the solvent. This suspension can then be measured by techniques such as PCS or laser diffraction.

Suitable methods to measure an accurate particle size where the active material has substantive aqueous solubility or the matrix has low solubility in a water based dispersant are outlined below.
1. In the circumstance where insoluble matrix such as microcrystalline cellulose prevents the measurement of the active material separation techniques such as filtration or centrifugation could be used to separate the insoluble matrix from the active material particles. Other ancillary techniques would also be required to determine if any active material was removed by the separation technique so that this could be taken into account.
2. In the case where the active material is too soluble in water other solvents could be evaluated for the measurement of particle size. Where a solvent could be found that active material is poorly soluble in but is a good solvent for the matrix a measurement would be relatively straight forward. If such a solvent is difficult to find another approach would be to measure the ensemble of matrix and active material in a solvent (such as isooctane) which both an insoluble in. Then the powder would be measured in another solvent where the active material is soluble but the matrix is not. Thus with a measurement of the matrix particle size and a measurement of the size of the matrix and active material together an understanding of the active material particle size can be obtained.
3. In some circumstances image analysis could be used to obtain information about the particle size distribution of the active material. Suitable image measurement techniques might include transmission electron microscopy (TEM), scanning electron microscopy (SEM), optical microscopy and confocal microscopy. In addition to these standard techniques some additional technique would be required to be used in parallel to differentiate the active material and matrix particles. Depending on the chemical makeup of the materials involved possible techniques could be elemental analysis, raman spectroscopy, FTIR spectroscopy or fluorescence spectroscopy.

### Other Definitions

Throughout this specification, unless the context requires otherwise, the phrase "dry mill" or variations, such as "dry milling", should be understood to refer to milling in at least the substantial absence of liquids. If liquids are present, they are present in such amounts that the contents of the mill retain the characteristics of a dry powder.

"Flowable" means a powder having physical characteristics rendering it suitable for further processing using typical equipment used for the manufacture of pharmaceutical compositions and formulations.

Other definitions for selected terms used herein may be found within the detailed description disclosed herein and apply throughout. Unless otherwise defined, all other scientific and technical terms used herein have the same meaning as commonly understood to one of ordinary skill in the art to which the invention belongs.

The term "millable" means that the grinding matrix is capable of being physically degraded under the dry milling conditions of the method disclosed herein. The milled grinding matrix can be of a comparable particle size to the biologically active material. The particle size of the matrix can be substantially reduced but not as small as the biologically active material

Other definitions for selected terms used herein may be found within the detailed description and apply throughout. Unless otherwise defined, all other scientific and technical terms used herein have the same meaning as commonly understood to one of ordinary skill in the art to which the invention belongs.

### Specific

Disclosed is a method for producing a composition, comprising the steps of: dry milling a solid biologically active material and a millable grinding matrix in a mill comprising a plurality of milling bodies, for a time period sufficient to produce particles of the biologically active material dispersed in an at least partially milled grinding material, wherein the composition produced by said method comprises particles of the biologically active compound at or above a volume fraction of 25 v/v%.

The mixture of active material and matrix may then be separated from the milling bodies and removed from the mill.

In one aspect the mixture of active material and matrix is then further processed. In another aspect, the grinding matrix is separated from the particles of biologically active material. In a further aspect, at least a portion of the milled grinding matrix is separated from the particulate biologically active material.

The milling bodies are essentially resistant to fracture and erosion in the dry milling process. The quantity of the grinding matrix relative to the quantity of biologically active material in particulate form, and the extent of milling of the grinding matrix, is sufficient to inhibit re-agglomeration of the particles of the active material.

The present description also relates to biologically active materials produced by said methods, to medicaments produced using said biologically active materials and to methods of treatment of an animal, including man, using a therapeutically effective amount of said biologically active materials administered by way of said medicaments.

### Increasing the volume fraction load

The present description is directed to the unexpected finding that particles of a biologically active material can be produced by dry milling processes wherein the composition produced by said method comprises particles of the biologically active compound at or above a volume fraction of 25 v/v%. In one surprising aspect the particle size produced by the process is equal to or less than 2000nm. In another surprising aspect the particle size produced by the process is equal to or less than 1000nm. This can result in a more efficient and cost effective process.

### Improving the dissolution profile

The process results in the biologically active material having an improved dissolution profile. An improved dissolution profile has significant advantages including the improvement of bioavailability of the biologically active material *in vivo.* The improved dissolution profile can be observed *in vitro.* Alternatively, the improved dissolution profile is observed *in vivo* by the observation of an improved bioavailability profile. Standard methods for determining the dissolution profile of a material *in vitro* are available in the art. A suitable method to determine an improved dissolution profile *in vitro* may include determining the concentration of the sample material in a solution over a period of time and comparing the results from the sample material to a control sample. An observation that peak solution concentration for the sample material was achieved in less time than the control sample would indicate (assuming it is statistically significant), that the sample material has an improved dissolution profile. The measurement sample is herein defined as the mixture of biologically active material with grinding matrix and/or other additives that has been subject to the processes described here. Herein a control sample is defined as a physical mixture (not subject to the processes described in this invention) of the components in the measurement sample with the same relative proportions of active, matrix and/or additive as the measurement sample. For the purposes of the dissolution testing a prototype formulation of the measurement sample could also be used. In this case the control sample would be formulated in the same way. Standard methods for determining the improved dissolution profile of a material *in vivo* are available in the art. A suitable method to determine an improved dissolution profile in a human may be after delivering the dose to measure the rate of active material absorption by measuring the plasma concentration of the sample compound over a period of time and comparing the results from the sample compound to a control. An observation that peak plasma concentration for the sample compound was achieved in less time than the control would indicate (assuming it is statistically significant) that the sample compound has improved bioavailability and an improved dissolution profile. The improved dissolution profile can be observed at a relevant gastrointestinal pH, when it is observed *in vitro.* The improved dissolution profile can be observed at a pH which is favourable at indicating improvements in dissolution when comparing the measurement sample to the control compound. Suitable methods for quantifying the concentration of a compound in an *in vitro* sample or an *in vivo* sample are widely available in the art. Suitable methods could include the use of spectroscopy or radioisotope labeling. The method of quantification of dissolution can be determined in a solution with a pH selected from the group consisting of: pH 1, pH 2, pH 3, pH 4, pH 5, pH 6, pH 7, pH 7.3, pH 7.4, pH 8, pH 9, pH 10, pH 11, pH 12, pH 13, pH 14 or a pH with 0.5 of a pH unit of any of this group.

### Crystallization Profile

Methods for determining the crystallinity profile of the biologically active material are widely available in the art. Suitable methods may include X-ray diffraction, differential scanning calorimetry, raman or IR spectrocopy.

### Amorphicity Profile

Methods for determining the amorphous content of the biologically active material are widely available in the art. Suitable methods may include X-ray diffraction, differential scanning calorimetry, raman or IR spectroscopy.

### Grinding Matrix

As will be described subsequently, selection of an appropriate grinding matrix affords particular advantageous applications of the method described herein.

A highly advantageous application of the method described herein is the use of a water-soluble grinding matrix in conjunction with a poorly water-soluble biologically active material. This affords at least two advantages. The first being when the powder containing the biologically active material is placed into water - such as the ingestion of the powder as part of an oral medication - the matrix dissolves, releasing the particulate active material such that there is maximum surface area exposed to solution, thereby allowing a rapid dissolution of the active compound. The second key advantage is the ability, if required, to remove or partially remove the matrix prior to further processing or formulation.

Another advantageous application of the method disclosed herein is the use of a water-insoluble grinding matrix, particularly in the area of agricultural use, when a biologically active material such as a fungicide is commonly delivered as part of a dry powder or a suspension. The presence of a water insoluble matrix will afford benefits such as increased rain fastness.

Without wishing to be bound by theory, it is believed that the physical degradation (including but not limited to particle size reduction) of the millable grinding matrix affords the advantage , by acting as a more effective diluent than grinding matrix of a larger particle size.

Again, as will be described subsequently, a highly advantageous aspect is that certain grinding matrixes appropriate for use in the method disclosed herein are also appropriate for use in a medicament. Disclosed are methods for the production of a medicament incorporating both the biologically active material and the grinding matrix or in some cases the biologically active material and a portion of the grinding matrix, medicaments so produced, and methods of treatment of an animal, including man, using a therapeutically effective amount of said biologically active materials by way of said medicaments.

Analogously, as will be described subsequently, a highly advantageous aspect is that certain grinding matrixes appropriate for use in the method disclosed herein are also appropriate for use in a carrier for an agricultural chemical, such as a pesticide, fungicide, or herbicide. Disclosed are methods for the production of an agricultural chemical composition incorporating both the biologically active material in particulate form and the grinding matrix, or in some cases the biologically active material, and a portion of the grinding matrix, and agricultural chemical compositions so produced. The medicament may include only the biologically active material together with the milled grinding matrix or the biologically active material and milled grinding matrix may be combined with one or more pharmaceutically acceptable carriers, as well as any desired excipients or other like agents commonly used in the preparation of medicaments. Analogously, the agricultural chemical composition may include only the biologically active material together with the milled grinding matrix or the biologically active materials and milled grinding matrix may be combined with one or more carriers, as well as any desired excipients or other like agents commonly used in the preparation of agricultural chemical compositions.

The grinding matrix can be both appropriate for use in a medicament and readily separable from the biologically active material by methods not dependent on particle size. Such grinding matrixes are described in the following detailed description. Such grinding matrixes are highly advantageous in that they afford significant flexibility in the extent to which the grinding matrix may be incorporated with the biologically active material into a medicament.

The grinding matrix can be harder than the biologically active material, and is thus capable of reducing the particle size of the active material under the dry milling conditions disclosed herein. Again without wishing to be bound by theory, under these circumstances it is believed that the millable grinding matrix affords the advantage through a second route, with the smaller particles of grinding matrix produced under the dry milling conditions enabling greater interaction with the biologically active material.

The quantity of the grinding matrix relative to the quantity of biologically active material, and the extent of physical degradation of the grinding matrix, is sufficient to improve to inhibit re-agglomeration of the particles of the active material. The quantity of the grinding matrix relative to the quantity of biologically active material, and the extent of physical degradation of the grinding matrix, can be sufficient to inhibit re-agglomeration of the particles of the active material in nanoparticulate form.

The grinding matrix is not generally selected to be chemically reactive with the biologically active material under the milling conditions disclosed herein, excepting for example, where the matrix is deliberately chosen to undergo a mechanico-chemical reaction. Such a reaction might be the conversion of a free base or acid to a salt or vice versa.

As stated above, the method disclosed herein requires the grinding matrix to be milled with the biologically active material; that is, the grinding matrix will physically degrade under the dry milling conditions disclosed herein to facilitate the formation and retention of particulates of the biologically active material with reduced particle size. The precise extent of degradation required will depend on certain properties of the grinding matrix and the biologically active material, the ratio of biologically active material to grinding matrix, and the particle size distribution of the particles comprising the biologically active material.

The physical properties of the grinding matrix necessary to achieve the requisite degradation are dependent on the precise milling conditions. For example, a harder grinding matrix may degrade to a sufficient extent provided it is subjected to more vigorous dry milling conditions.

Physical properties of the grinding matrix relevant to the extent that the agent will degrade under dry milling conditions include hardness, friability, as measured by indicia such as hardness, fracture toughness and brittleness index.

A low hardness (typically a Mohs Hardness less than 7) of the biologically active material is desirable to ensure fracture of the particles during processing, so that composite microstructures develop during milling. The hardness can be less than 3 as determined using the Mohs Hardness scale.

The grinding matrix can be of low abrasivity. Low abrasivity is desirable to minimise contamination of the mixture of the biologically active material in the grinding matrix by the milling bodies and/or the milling chamber of the media mill. An indirect indication of the abrasivity can be obtained by measuring the level of milling-based contaminants.

The grinding matrix can have a low tendency to agglomerate during dry milling. While it is difficult to objectively quantify the tendency to agglomerate during milling, it is possible to obtain a subjective measure by observing the level of "caking" of the grinding matrix on the milling bodies and the milling chamber of the media mill as dry milling progresses.

The grinding matrix may be an inorganic or organic substance.

In one embodiment, the grinding matrix is selected from the following, either as a single substance or a combination of two or more substances: Polyols (sugar alcohols) for example (but not limited to) mannitol, sorbitol, isomalt, xylitol, maltitol, lactitol, erythritol, arabitol, ribitol, monosaccharides for example (but not limited to) glucose, fructose, mannose, galactose, disaccharides and trisaccharides for example (but not limited to) anhydrous lactose, lactose monohydrate, sucrose, maltose, trehalose, polysaccharides for example (but not limited to) maltodextrins, dextrin, Inulin, dextrates, polydextrose, other carbohyrates for example (but not limited to) starch, wheat flour, corn flour, rice flour, rice starch, tapioca flour, tapioca starch, potato flour, potato starch, other flours and starches, , soy flour, soy meal or other soy products, cellulose, microcrystalline cellulose, microcrystalline cellulose based co blended excipients, chemically modified excipients such as pregelatinized (or partially) starch, modified celluloses such as HPMC, CMC, HPC, enteric polymer coatings such as hypromellose phthalate, cellulose acetate phthalate (Aquacoat®), polyvinyl acetate phthalate (Sureteric®), hypromellose acetate succinate (AQOAT®), and polmethacrylates (Eudragit® and Acryl-EZE®), Milk products for example (but not limited to) milk powder, skim milk powders, other milk solids and dreviatives, other functional Excipients, organic acids for example (but not limited to) citric acid, tartaric acid, malic acid, maleic acid fumaric acid , ascorbic acid, succinic acid, the conjugate salt of organic acids for example (but not limited to) sodium citrate, sodium tartrate, sodium malate, sodium ascorbate, potassium citrate, potassium tartrate, potassium malate, potassium ascorbate, inorganicls such as sodium carbonate, potassium carbonate, magnesium carbonate, sodium bicarbonate, potassium bicarbonate and calcium carbonate. dibasic calcium phosphate, tribasic calcium phosphate, sodium sulfate, sodium chloride, sodium metabisulphite, sodium thiosulfate, ammonium chloride, Glauber's salt, ammonium carbonate, sodium bisulfate, magnesium sulfate, potash alum, potassium chloride, sodium hydrogen sulfate, sodium hydroxide, crystalline hydroxides, hydrogen carbonates, hydrogen carbonates of pharmaceutical acceptable alkali metals, such as but not limited by, sodium, potassium, lithium, calcium, and barium, ammonium salts (or salts of volatile amines), for example (but not limited to) ammonium chloride, methylamine hydrochloride, ammonium bromide, other inorganics for example (but not limited to), thermal silica, chalk, mica, silica, alumina, titanium dioxide, talc, kaolin, bentonite, hectorite, magnesium trisilicate, other clay or clay derivatives or aluminium silicates, a surfactant for example (but not limited to) sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, poloxamer 407, poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines.

The grinding matrix can be a matrix that is considered GRAS (generally regarded as safe) by persons skilled in the pharmaceutical arts.

A combination of two or more suitable matrices, such as those listed above, can be used as the grinding matrix to provide improved properties such as the reduction of caking, and greater improvement of particle size reduction. Combination matrices may also be advantageous when the matrices have different solubility's allowing the removal or partial removal of one matrix, while leaving the other or part of the other to provide encapsulation or partial encapsulation of the biologically active material.

Another aspect of the method is the inclusion of a suitable milling aid in the matrix to improve milling performance. Improvements to milling performance would be things such as, but not limited to, a reduction in caking or higher recovery of powder from the mill. Examples of suitable milling aids include surfactants, polymers and inorganics such as silica (including colloidal silica), aluminium silicates and clays.

There are a wide range of surfactants that will make suitable milling aids. The highly preferred form is where the surfactant is in a solid, or can be manufactured into a solid. The surfactant can be selected from the group consisting of: polyoxyethylene alkyl ethers, polyoxyethylene stearates, polyethylene glycols (PEG), poloxamers, poloxamines, sarcosine based surfactants, polysorbates, aliphatic alcohols, alkyl and aryl sulfates, alkyl and aryl polyether sulfonates and other sulfate surfactants, trimethyl ammonium based surfactants, lecithin and other phospholipids, bile salts, polyoxyethylene castor oil derivatives, polyoxyethylene sorbitan fatty acid esters, Sorbitan fatty acid esters, Sucrose fatty acid esters, alkyl glucopyranosides, alkyl maltopyranosides, glycerol fatty acid esters, Alkyl Benzene Sulphonic Acids, Alkyl Ether Carboxylic Acids, Alkyl and aryl Phosphate esters, Alkyl and aryl Sulphate esters, Alkyl and aryl Sulphonic acids, Alkyl Phenol Phosphates esters, Alkyl Phenol Sulphates esters, Alkyl and Aryl Phosphates, Alkyl Polysaccharides, Alkylamine Ethoxylates, Alkyl-Naphthalene Sulphonates formaldehyde condensates, Sulfosuccinates, lignosulfonates, Ceto-Oleyl Alcohol Ethoxylates, Condensed Naphthalene Sulphonates, Dialkyl and Alkyl Naphthalene Sulphonates,Di-alkyl Sulphosuccinates, Ethoxylated nonylphenols, Ethylene Glycol Esters,Fatty Alcohol Alkoxylates, Hydrogenated tallowalkylamines, Mono-alkyl Sulphosuccinamates, Nonyl Phenol Ethoxylates, Sodium Oleyl N-methyl Taurate, Tallowalkylamines, linear and branched dodecylbenzene sulfonic acids. The surfactant can be selected from the group consisting of: sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate, glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, poloxamer 407, poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines.

The polymer can be selected from the list of: polyvinylpyrrolidones (PVP), polyvinylalcohol, Acrylic acid based polymers and copolymers of acrylic acid

The milling aid can have a concentration selected from the group consisting of: 0.1 -10 % w/w, 0.1 -5% w/w, 0.1 -2.5% w/w, of 0.1 - 2% w/w, 0.1 -1 %, 0.5-5% w/w, 0.5 -3% w/w, 0.5 -2% w/w, 0.5 - 1.5%, 0.5 -1 % w/w, of 0.75 - 1.25 % w/w, 0.75 -1% and 1% w/w.

### Milling bodies

In the method dislcosed herein, the milling bodies can be chemically inert and rigid. The term "chemically-inert", as used herein, means that the milling bodies do not react chemically with the biologically active material or the grinding matrix.

As described above, the milling bodies are essentially resistant to fracture and erosion in the milling process.

The milling bodies are desirably provided in the form of bodies which may have any of a variety of smooth, regular shapes, flat or curved surfaces, and lacking sharp or raised edges. For example, suitable milling bodies can be in the form of bodies having ellipsoidal, ovoid, spherical or right cylindrical shapes. The milling bodies can be provided in the form of one or more of beads, balls, spheres, rods, right cylinders, drums or radius-end right cylinders (i.e., right cylinders having hemispherical bases with the same radius as the cylinder).

Depending on the nature of the biologically active material and the grinding matrix, the milling media bodies desirably have an effective mean particle diameter (i.e. "particle size") between about 0.1 and 30 mm, between about 1 and about 15 mm, or between about 3 and 10 mm. The milling bodies may comprise various substances such as ceramic, glass, metal or polymeric compositions, in a particulate form. Suitable metal milling bodies are typically spherical and generally have good hardness (i.e. RHC 60-70), roundness, high wear resistance, and narrow size distribution and can include, for example, balls fabricated from type 52100 chrome steel, type 316 or 440C stainless steel or type 1065 high carbon steel.

Ceramics, for example, can be selected from a wide array of ceramics desirably having sufficient hardness and resistance to fracture to enable them to avoid being chipped or crushed during milling and also having sufficiently high density. Suitable densities for milling media can range from about 1 to 15 g/cm^{3'}, or from about 1 to 8 g/cm³. Ceramics can be selected from steatite, aluminum oxide, zirconium oxide, zirconia-silica, yttria-stabilized zirconium oxide, magnesia-stabilized zirconium oxide, silicon nitride, silicon carbide, cobalt-stabilized tungsten carbide, and the like, as well as mixtures thereof.

Glass milling media can be spherical (e.g. beads), have a narrow size distribution, are durable, and include, for example, lead-free soda lime glass and borosilicate glass. Polymeric milling media can be substantially spherical and can be selected from a wide array of polymeric resins having sufficient hardness and friability to enable them to avoid being chipped or crushed during milling, abrasion-resistance to minimize attrition resulting in contamination of the product, and freedom from impurities such as metals, solvents, and residual monomers.

P resins, for example, can be selected from crosslinked polystyrenes, such as polystyrene crosslinked with divinylbenzene, styrene copolymers, polyacrylates such as polymethylmethacrylate, polycarbonates, polyacetals, vinyl chloride polymers and copolymers, polyurethanes, polyamides, high density polyethylenes, polypropylenes, and the like. The use of polymeric milling media to grind materials down to a very small particle size (as opposed to mechanochemical synthesis) is disclosed, for example, in U.S. patents 5,478,705 and 5,500,331. Polymeric resins typically can have densities ranging from about 0.8 to 3.0 g/cm³. Higher density polymeric resins are preferred. Alternatively, the milling media can be composite particles comprising dense core particles having a polymeric resin adhered thereon. Core particles can be selected from substances known to be useful as milling media, for example, glass, alumina, zirconia silica, zirconium oxide, stainless steel, and the like. Core substances can have densities greater than about 2.5 g/cm³.

The milling media can be formed from a ferromagnetic substance, thereby facilitating removal of contaminants arising from wear of the milling media by the use of magnetic separation techniques.

Each type of milling body has its own advantages. For example, metals have the highest specific gravities, which increase grinding efficiency due to increased impact energy. Metal costs range from low to high, but metal contamination of final product can be an issue. Glasses are advantageous from the standpoint of low cost and the availability of small bead sizes as low as 0.004 mm. However, the specific gravity of glasses is lower than other media and significantly more milling time is required. Finally, ceramics are advantageous from the standpoint of low wear and contamination, ease of cleaning, and high hardness.

### Dry Milling

In the dry milling process disclosed herein, the biologically active material and grinding matrix, in the form of crystals, powders, or the like, are combined in suitable proportions with the plurality of milling bodies in a milling chamber that is mechanically agitated (i.e. with or without stirring) for a predetermined period of time at a predetermined intensity of agitation. Typically, a milling apparatus is used to impart motion to the milling bodies by the external application of agitation, whereby various translational, rotational or inversion motions or combinations thereof are applied to the milling chamber and its contents, or by the internal application of agitation through a rotating shaft terminating in a blade, propeller, impeller or paddle or by a combination of both actions.

During milling, motion imparted to the milling bodies can result in application of shearing forces as well as multiple impacts or collisions having significant intensity between milling bodies and particles of the biologically active material and grinding matrix. The nature and intensity of the forces applied by the milling bodies to the biologically active material and the grinding matrix is influenced by a wide variety of processing parameters including: the type of milling apparatus; the intensity of the forces generated, the kinematic aspects of the process; the size, density, shape, and composition of the milling bodies; the weight ratio of the biologically active material and grinding matrix mixture to the milling bodies; the duration of milling; the physical properties of both the biologically active material and the grinding matrix; the atmosphere present during activation; and others.

Advantageously, the media mill is capable of repeatedly or continuously applying mechanical compressive forces and shear stress to the biologically active material and the grinding matrix. Suitable media mills include but are not limited to the following: high-energy ball, sand, bead or pearl mills, basket mill, planetary mill, vibratory action ball mill, multi-axial shaker/mixer, stirred ball mill, horizontal small media mill, multi-ring pulverizing mill, and the like, including small milling media. The milling apparatus also can contain one or more rotating shafts.

TThe dry milling can be a ball mill. Throughout the remainder of the specification reference will be made to dry milling being carried out by way of a ball mill. Examples of this type of mill are attritor mills, nutating mills, tower mills, planetary mills, vibratory mills and gravity-dependent-type ball mills. It will be appreciated that dry milling in accordance with the method dislcosed herein may also be achieved by any suitable means other than ball milling. For example, dry milling may also be achieved using jet mills, rod mills, roller mills or crusher mills.

### Biologically active material

The biologically active material includes active compounds, including compounds for veterinary and human use such as but not limited to, pharmaceutical actives, neutraceuticals, cosmeceuticals, cosmetics, complementary medicines, natural products, vitamins, nutrients, biologics, amino acids, proteins, peptides, nucleotides, nucleic acids, and agricultural compounds such as pesticides, herbicides and fungicides, germinating agents and the like.

Other biologically active materials include, but are not limited to, foods, seeds, cocoa cocoa powder, cocoa nibs, cocoa mass, cocoa liquor, cocoa solids, coffee, herbs, spices, other plant materials, minerals, animal products, shells and other skeletal material.

The biologically active material can be an organic compound. In a highly red form of the , the biologically active material is an organic, therapeutically active compounds for veterinary or human use.

The biologically active material can be an inorganic compound. The biologically active material can be sulphur, copper hydroxide, an organometallic complex or copper oxychloride.

The biologically active material is ordinarily a material for which one of skill in the art desires improved dissolution properties. The biologically active material may be a conventional active agent or drug, although the process disclosed herein may be employed on formulations or agents that already have reduced particle size compared to their conventional form.

Biologically active materials suitable for use in the method include actives, biologics, amino acids, proteins, peptides, nucleotides, nucleic acids, and analogs, homologs and first order derivatives thereof. The biologically active material can be selected from a variety of known classes of drugs, including, but not limited to: anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, cough suppressants (expectorants and mucolytics), diagnostic agents, diagnostic imaging agents, diuretics, dopaminergics (anti-Parkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines.

A description of these classes of active agents and a listing of species within each class can be found in Martindale's The Extra Pharmacopoeia, 31st Edition (The Pharmaceutical Press, London, 1996). Another source of active agents is the Physicians Desk Reference (60th Ed., pub. 2005), familiar to those of skill in the art. The active agents are commercially available and/or can be prepared by techniques known in the art.

An exhaustive list of drugs for which the methods disclosed herein are suitable would be burdensomely long for this specification; however, reference to the general pharmacopoeia listed above would allow one of skill in the art to select virtually any drug to which the method disclosed herein may be applied.

In addition it is also expected that new chemical entities (NCE) and other actives for which the methods disclosed herein are suitable will be created or become commercially in the future.

Notwithstanding the general applicability of the method disclosed herein, more specific examples of biologically active materials include, but are not limited to: haloperidol (dopamine antagonist), DL isoproterenol hydrochloride (β-adrenergic agonist), terfenadine (H1-antagonist), propranolol hydrochloride (β-adrenergic antagonist), desipramine hydrochloride (antidepressant), sildenafil citrate, tadalafil and vardenafil. Minor analgesics (cyclooxygenase inhibitors), fenamic acids, Piroxicam, Cox-2 inhibitors, and Naproxen, and others, may all benefit from being prepared. As discussed in the context of the background to the invention, biologically active materials that are poorly water soluble at gastrointestinal pH will particularly benefit from being prepared, and the method disclosed herein is particularly advantageously applied to materials that are poorly water soluble at gastrointestinal pH.

Such materials include, but are not limited to: albendazole, albendazole sulfoxide, alfaxalone, acetyl digoxin, acyclovir analogs, alprostadil, aminofostin, anipamil, antithrombin III, atenolol, azidothymidine, beclobrate, beclomethasone, belomycin, benzocaine and derivatives, beta carotene, beta endorphin, beta interferon, bezafibrate, binovum, biperiden, bromazepam, bromocryptine, bucindolol, buflomedil, bupivacaine, busulfan, cadralazine, camptothesin, canthaxanthin, captopril, carbamazepine, carboprost, cefalexin, cefalotin, cefamandole, cefazedone, cefluoroxime, cefinenoxime, cefoperazone, cefotaxime, cefoxitin, cefsulodin, ceftizoxime, chlorambucil, chromoglycinic acid, ciclonicate, ciglitazone, clonidine, cortexolone, corticosterone, cortisol, cortisone, cyclophosphamide, cyclosporin A and other cyclosporins, cytarabine, desocryptin, desogestrel, dexamethasone esters such as the acetate, dezocine, diazepam, diclofenac, dideoxyadenosine, dideoxyinosine, digitoxin, digoxin, dihydroergotamine, dihydroergotoxin, diltiazem, dopamine antagonists, doxorubicin, econazole, endralazine, enkephalin, enalapril, epoprostenol, estradiol, estramustine, etofibrate, etoposide, factor ix, factor viii, felbamate, fenbendazole, fenofibrate, fexofenedine, flunarizin, flurbiprofen, 5-fluorouracil, flurazepam, fosfomycin, fosmidomycin, furosemide, gallopamil, gamma interferon, gentamicin, gepefrine, gliclazide, glipizide, griseofulvin, haptoglobulin, hepatitis B vaccine, hydralazine, hydrochlorothiazide, hydrocortisone, ibuprofen, ibuproxam, indinavir, indomethacin, iodinated aromatic x-ray contrast agents such as iodamide, ipratropium bromide, ketoconazole, ketoprofen, ketotifen, ketotifen fumarate, K-strophanthin, labetalol, lactobacillus vaccine, lidocaine, lidoflazin, lisuride, lisuride hydrogen maleate, lorazepam, lovastatin, mefenamic acid, melphalan, memantin, mesulergin, metergoline, methotrexate, methyl digoxin, methylprednisolone, metronidazole, metisoprenol, metipranolol, metkephamide, metolazone, metoprolol, metoprolol tartrate, miconazole, miconazole nitrate, minoxidil, misonidazol, molsidomin, nadolol, nafiverine, nafazatrom, naproxen, natural insulins, nesapidil, nicardipine, nicorandil, nifedipine, niludipin, nimodipine, nitrazepam, nitrendipine, nitrocamptothesin, 9-nitrocamptothesin, olanzapine, oxazepam, oxprenolol, oxytetracycline, penicillins such as penicillin G benethamine, penecillin O, phenylbutazone, picotamide, pindolol, piposulfan, piretanide, piribedil, piroxicam, pirprofen, plasminogenici activator, prednisolone, prednisone, pregnenolone, procarbacin, procaterol, progesterone, proinsulin, propafenone, propanolol, propentofyllin, propofol, propranolol, raloxifene, rifapentin, simvastatin, semi-synthetic insulins, sobrerol, somastotine and its derivatives, somatropin, stilamine, sulfinalol hydrochloride, sulfinpyrazone, suloctidil, suprofen, sulproston, synthetic insulins, talinolol, taxol, taxotere, testosterone, testosterone propionate, testosterone undecanoate, tetracane HI, tiaramide HCl, tolmetin, tranilast, triquilar, tromantadine HCl, urokinase, valium, verapamil, vidarabine, vidarabine phosphate sodium salt, vinblastine, vinburin, vincamine, vincristine, vindesine, vinpocetine, vitamin A, vitamin E succinate, and x-ray contrast agents. Drugs can be neutral species or basic or acidic as well as salts of an acid or base. Specifically the chemical makeup and the functional groups, including an acid or base group, are generally not the determinant factor, excepting a possible chemical reaction with a specific matrix, for the successful creation of a biologically active substance with reduced particle size. This method disclosed herein is not limited to any drug specific class, application type, chemical type or function grouping. Rather the suitability of a biologically active material for use in this method disclosed herein is primarily determined by the mechanical properties of the material. In addition, some biologically active materials may have the benefit of absorption through the skin if presented in a particle formulation. Such biologically active materials include, but are not limited to, Voltaren (diclofenac), rofecoxib, and ibuprofen.

Conveniently, the biologically active material is capable of withstanding temperatures that are typical in uncooled dry milling, which may exceed 80°C. Therefore, materials with a melting point about 80 °C or greater are highly suitable. For biologically active materials with lower melting points, the media mill may be cooled, thereby allowing materials with significantly lower melting temperatures to be processed according to the method disclosed herein. For instance, a simple water-cooled mill will keep temperatures below 50 °C, or chilled water could be used to further lower the milling temperature. Those skilled in the art will understand that a high energy ball mill could be designed to run at any temperature between say -30 to 200 °C. For some biologically active materials it may be advantageous to control the milling temperature to temperatures significantly below the melting points of the biologically active materials.

The biologically active material is obtained in a conventional form commercially and/or prepared by techniques known in the art.

It is preferred, but not essential, that the particle size of the biologically active material be less than about 1000 µm, as determined by sieve analysis. If the coarse particle size of the biologically active material is greater than about 1000µm, then it is preferred that the particles of the biologically active material substrate be reduced in size to less than 1000 µm using a another standard milling method.

### Processed biologically active material

The biologically active materials, which have been subject to the methods disclosed herein , can comprise particles of biologically active material of an average particle size, determined on a particle number basis, is equal to or less than a size selected from the group consisting of: 10,000nm, 8000nm, 6000nm, 5000nm, 4000nm, 3000nm, 2000nm, 1900nm, 1800nm, 1700nm, 1600nm, 1500nm, 1400nm, 1300nm, 1200nm, 1100nm, 1000nm, 900nm, 800nm, 700nm, 600nm, 500nm, 400nm, 300nm, 200nm and 100 nm.

The biologically active materials, which have been subject to the methods disclosed herein, can comprise particles of biologically active material of a median particle size, determined on a particle volume basis, equal or less than a size selected from the group consisting of: 20000nm, 15000nm, 10000nm, 7500nm, 5000nm, 2000nm, 1900nm, 1800nm, 1700nm, 1600nm, 1500nm, 1400nm, 1300nm, 1200nm, 1100nm, 1000nm, 900nm, 800nm, 700nm, 600nm, 500nm, 400nm, 300nm, 200nm and 100nm.

The biologically active materials, which have been subject to the methods disclosed herein, comprises particles of biologically active material and wherein the Dx of the particle size distribution, as measured on a particle volume basis, is selected from the group consisting of less than or equal to 10,000nm, 5000nm, 3000nm, 2000nm, 1900nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm, and 100nm; wherein x is greater than or equal to 90,

These sizes refer to particles either fully dispersed or partially agglomerated.

### Agglomerates of biologically active material after processing

Agglomerates comprising particles of biologically active material, said particles having a particle size within the ranges specified above, should be understood to fall within the scope of the present disclosure, regardless of whether the agglomerates exceed the ranges specified above. Agglomerates comprising particles of biologically active material, said agglomerates having a total agglomerate size within the ranges specified above, should be understood to fall within the scope of the present disclosure.

Agglomerates comprising particles of biologically active material, should be understood to fall within the scope of the present disclosure if at the time of use, or further processing, the particle size of the agglomerate is within the ranges specified above.

Agglomerates comprising particles of biologically active material, said particles having a particle size within the ranges specified above, at the time of use, or further processing, should be understood to fall within the scope of the present disclosure, regardless of whether the agglomerates exceed the ranges specified above.

### Processing Time

The biologically active material and the grinding matrix can be dry milled for the shortest time necessary to form the mixture of the biologically active material in the grinding matrix such that the active material has improved dissolution to minimise any possible contamination from the media mill and/or the plurality of milling bodies. This time varies greatly, depending on the biologically active material and the grinding matrix, and may range from as short as 1 minute to several hours. Dry milling times in excess of 2 hours may lead to degradation of the biologically active material and an increased level of undesirable contaminants.

Suitable rates of agitation and total milling times are adjusted for the type and size of milling apparatus as well as the milling media, the weight ratio of the biologically active material and grinding matrix mixture to the plurality of milling bodies, the chemical and physical properties of the biologically active material and grinding matrix, and other parameters that may be optimized empirically.

### Inclusion of the grinding matrix with the biologically active material and separation of the grinding matrix from the biologically active material

The grinding matrix may be not separated from the biologically active material but is maintained with the biologically active material in the final product. The grinding matrix can be considered to be Generally Regarded as Safe (GRAS) for pharmaceutical products.

In an alternative aspect, the grinding matrix is separated from the biologically active material. In one aspect, where the grinding matrix is not fully milled, the unmilled grinding matrix is separated from the biologically active material. In a further aspect, at least a portion of the milled grinding matrix is separated from the biologically active material.

Any portion of the grinding matrix may be removed, including but not limited to 10%, 25%, 50%, 75%, or substantially all of the grinding matrix.

A significant portion of the milled grinding matrix may comprise particles of a size similar to and/or smaller than the particles comprising the biologically active material. Where the portion of the milled grinding matrix to be separated from the particles comprising the biologically active material comprises particles of a size similar to and/or smaller than the particles comprising the biologically active material, separation techniques based on size distribution are inapplicable.

In these circumstances, the method disclosed herein may involve separation of at least a portion of the milled grinding matrix from the biologically active material by techniques including but not limited to electrostatic separation, magnetic separation, centrifugation (density separation), hydrodynamic separation, froth flotation.

Advantageously, the step of removing at least a portion of the milled grinding matrix from the biologically active material may be performed through means such as selective dissolution, washing, or sublimation.

An advantageous aspect of the method disclosed herein would be the use of grinding matrix that has two or more components where at least one component is water soluble and at least one component has low solubility in water. In this case washing can be used to remove the matrix component soluble in water leaving the biologically active material encapsulated in the remaining matrix components. In a highly advantageous aspect of the method disclosed herein the matrix with low solubility is a functional excipient.

A highly advantageous aspect of the present method disclosed herein is that certain grinding matrixes appropriate for use in the method of the (in that they physically degrade to the desired extent under dry milling conditions) are also pharmaceutically acceptable and thus appropriate for use in a medicament. Where the method disclosed herein does not involve complete separation of the grinding matrix from the biologically active material, the present method disclosed herein encompasses methods for the production of a medicament incorporating both the biologically active material and at least a portion of the milled grinding matrix, medicaments so produced and methods of treatment of an animal, including man, using a therapeutically effective amount of said biologically active materials by way of said medicaments.

The medicament may include only the biologically active material and the grinding matrix or the biologically active materials and grinding matrix may be combined with one or more pharmaceutically acceptable carriers, as well as any desired excipients or other like agents commonly used in the preparation of medicaments.

Analogously, a highly advantageous aspect of the present method disclosed herein is that certain grinding matrixes appropriate for use in the method disclosed herein (in that they physically degrade to a desirable extent under dry milling conditions) are also appropriate for use in an agricultural chemical composition. Where the method disclosed herein does not involve complete separation of the grinding matrix from the biologically active material, the present method disclosed herein encompasses methods for the production of a agricultural chemical composition incorporating both the biologically active material and at least a portion of the milled grinding matrix, agricultural chemical composition so produced and methods of use of such compositions.

The agricultural chemical composition may include only the biologically active material and the grinding matrix or the biologically active materials and grinding matrix may be combined with one or more acceptable carriers, as well as any desired excipients or other like agents commonly used in the preparation of agricultural chemical compositions.

The grinding matrix can be both appropriate for use in a medicament and readily separable from the biologically active material by methods not dependent on particle size. Such grinding matrixes are described in the following detailed description. Such grinding matrixes are highly advantageous in that they afford significant flexibility in the extent to which the grinding matrix may be incorporated with the biologically active material into a medicament.

The mixture of biologically active material and grinding matrix may then be separated from the milling bodies and removed from the mill.

In one embodiment, the grinding matrix is separated from the mixture of biologically active material and grinding matrix. Where the grinding matrix is not fully milled, the unmilled grinding matrix is separated from the biologically active material. In a further aspect, at least a portion of the milled grinding matrix is separated from the biologically active material.

The milling bodies are essentially resistant to fracture and erosion in the dry milling process. The quantity of the grinding matrix relative to the quantity of biologically active material, and the extent of milling of the grinding matrix, is sufficient to provide reduced particle size of the biologically active material.

The grinding matrix is not chemically nor mechanically reactive with the pharmaceutical material under the dry milling conditions of the method disclosed herein except, for example, where the matrix is deliberately chosen to undergo a mechanico-chemical reaction. Such a reaction might be the conversion of a free base or acid to a salt or vice versa.

The medicament can be a solid dosage form, however, other dosage forms may be prepared by those of ordinary skill in the art.

In one form, after the step of separating said mixture of biologically active material and grinding matrix from the plurality of milling bodies, and before the step of using said mixture of biologically active material and grinding matrix in the manufacture of a medicament, the method may comprise the step of:
removing a portion of the grinding matrix from said mixture of biologically active material and grinding matrix to provide a mixture enriched in the biologically active material;
and the step of using said mixture of biologically active material and grinding matrix in the manufacture of a medicament, more particularly comprises the step of using the mixture of biologically active material and grinding matrix enriched in the biologically active material form in the manufacture of a medicament.

Disclosed medicaments manufactured by said methods, and methods for the treatment of an animal, including man, by the administration of a therapeutically effective amount of the biologically active materials by way of said medicaments.

A facilitating agent or a combination of facilitating agents can also be comprised in the mixture to be milled. Such facilitating agents appropriate for use in the method disclosed herein include diluents, surfactants, polymers, binding agents, filling agents, lubricating agents, sweeteners, flavouring agents, preservatives, buffers, wetting agents, disintegrants, effervescent agents and agents that may form part of a medicament, including a solid dosage form, or other excipients required for other specific drug delivery, such as the agents and media listed below under the heading *Medicinal and Pharmaceutical Compositions,* or any combination thereof.

### Biologically active materials and compositions

Disclosed are pharmaceutically acceptable materials produced according to the methods disclosed herein, compositions including such materials, including compositions comprising such materials together with the grinding matrix with or without milling aids, fascilitating agents, with at least a portion of the grinding matrix or separated from the grinding matrix.

The pharmaceutically acceptable materials within the compositions disclosed herein are present at a concentration of between about 0.1 % and about 99.0% by weight. The concentration of pharmaceutically acceptable materials within the compositions can be about 5% to about 80% by weight, while concentrations of 10% to about 50% by weight are also disclosed. Desirably, the concentration will be in the range of about 10 to 15% by weight, 15 to 20% by weight, 20 to 25% by weight, 25 to 30% by weight, 30 to 35% by weight, 35 to 40% by weight, 40 to 45% by weight, 45 to 50% by weight, 50 to 55% by weight, 55 to 60% by weight, 60 to 65% by weight, 65 to 70% by weight, 70 to 75% by weight or 75 to 80% by weight for the composition prior to any later removal (if desired) of any portion of the grinding matrix. Where part or the entire grinding matrix has been removed, the relative concentration of pharmaceutically acceptable materials in the composition may be considerably higher depending on the amount of the grinding matrix that is removed. For example, if the entire grinding matrix is removed the concentration of particles in the preparation may approach 100% by weight (subject to the presence of facilitating agents).

Compositions produced according to the method disclosed herein are not limited to the inclusion of a single species of pharmaceutically acceptable materials. More than one species of pharmaceutically acceptable materials may therefore be present in the composition. Where more than one species of pharmaceutically acceptable materials is present, the composition so formed may either be prepared in a dry milling step, or the pharmaceutically acceptable materials may be prepared separately and then combined to form a single composition.

### Medicaments

Disclosed are pharmaceutically acceptable material, optionally together with the grinding matrix or at least a portion of the grinding matrix, with or without milling aids, fascilitating agents, combined with one or more pharmaceutically acceptable carriers, as well as other agents commonly used in the preparation of pharmaceutically acceptable compositions.

As used herein "pharmaceutically acceptable carrier" includes any and all solvents, dispersion media, coatings, antibacterial and antifungal agents, isotonic and absorption delaying agents, and the like that are physiologically compatible. The carrier can be suitable for parenteral administration, intravenous, intraperitoneal, intramuscular, sublingual, pulmonary, transdermal or oral administration. Pharmaceutically acceptable carriers include sterile aqueous solutions or dispersions and sterile powders for the extemporaneous preparation of sterile injectable solutions or dispersion. The use of such media and agents for the manufacture of medicaments is well known in the art. Except insofar as any conventional media or agent is incompatible with the pharmaceutically acceptable material, use thereof in the manufacture of a pharmaceutical composition according to the method disclosed herein is contemplated.

Pharmaceutical acceptable carriers may include one or more of the following examples:
(1) surfactants and polymers" including, but not limited to polyethylene glycol (PEG), polyvinylpyrrolidone (PVP), polyvinylalcohol, crospovidone, polyvinylpyrrolidone-polyvinylacrylate copolymer, cellulose derivatives, hydroxypropylmethyl cellulose, hydroxypropyl cellulose, carboxymethylethyl cellulose, hydroxypropyllmethyl cellulose phthalate, polyacrylates and polymethacrylates, urea, sugars, polyols, and their polymers, emulsifiers, sugar gum, starch, organic acids and their salts, vinyl pyrrolidone and vinyl acetate; and or
(2) binding agents such as various celluloses and cross-linked polyvinylpyrrolidone, microcrystalline cellulose; and or
(3) filling agents such as lactose monohydrate, lactose anhydrous, microcrystalline cellulose and various starches; and or
(4) lubricating agents such as agents that act on the flowability of the powder to be compressed, including colloidal silicon dioxide, talc, stearic acid, magnesium stearate, calcium stearate, silica gel; and or
(5) sweeteners such as any natural or artificial sweetener including sucrose, xylitol, sodium saccharin, cyclamate, aspartame, and accsulfame K; and or
(6) flavouring agents; and or
(7) preservatives such as potassium sorbate, methylparaben, propylparaben, benzoic acid and its salts, other esters of parahydroxybenzoic acid such as butylparaben, alcohols such as ethyl or benzyl alcohol, phenolic chemicals such as phenol, or quarternary compounds such as benzalkonium chloride; and or
(8) buffers; and or
(9) Diluents such as pharmaceutically acceptable inert fillers, such as microcrystalline cellulose, lactose, dibasic calcium phosphate, saccharides, and/or mixtures of any of the foregoing; and or
(10) wetting agents such as corn starch, potato starch, maize starch, and modified starches, croscarmellose sodium, crosspovidone, sodium starch glycolate, and mixtures thereof; and or
(11) disintegrants; and or
(12) effervescent agents such as effervescent couples such as an organic acid (e.g., citric, tartaric, malic, fumaric, adipic, succinic, and alginic acids and anhydrides and acid salts), or a carbonate (e.g. sodium carbonate, potassium carbonate, magnesium carbonate, sodium glycine carbonate, L-lysine carbonate, and arginine carbonate) or bicarbonate (e.g. sodium bicarbonate or potassium bicarbonate); and or
(13) other pharmaceutically acceptable excipients.

Medicaments disclosed herein suitable for use in animals and in particular in man typically must be sterile and stable under the conditions of manufacture and storage. The medicaments disclosed herein comprising the biologically active material can be formulated as a solid, a solution, a microemulsion, a liposome, or other ordered structures suitable to high drug concentration. Actual dosage levels of the biologically active material in the medicament disclosed herein may be varied in accordance with the nature of the biologically active material, as well as the potential increased efficacy due to the advantages of providing and administering the biologically active material (e.g., increased solubility, more rapid dissolution, increased surface area of the biologically active material, etc.). Thus as used herein "therapeutically effective amount" will refer to an amount of biologically active material required to effect a therapeutic response in an animal. Amounts effective for such a use will depend on: the desired therapeutic effect; the route of administration; the potency of the biologically active material; the desired duration of treatment; the stage and severity of the disease being treated; the weight and general state of health of the patient; and the judgment of the prescribing physician.

In another embodiment, the biologically active material, optionally together with the grinding matrix or at least a portion of the grinding matrix, disclosed herein may be combined into a medicament with another biologically active material, or even the same biologically active material. In the latter embodiment, a medicament may be achieved which provides for different release characteristics - early release from the biologically active material, and later release from a larger average size biologically active material.

### Modes of administration of medicaments comprising biologically active materials

Medicaments disclosed herein can be administered to animals, including man, in any pharmaceutically acceptable manner, such as orally, rectally, pulmonary, intravaginally, locally (powders, ointments or drops), transdermal, parenteral administration, intravenous, intraperitoneal, intramuscular, sublingual or as a buccal or nasal spray.

Solid dosage forms for oral administration include capsules, tablets, pills, powders, pellets, and granules. Further, incorporating any of the normally employed excipients, such as those previously listed, and generally 5-95% of the biologically active agent, and at a concentration of 10%-75% will form a pharmaceutically acceptable non-toxic oral composition.

Medicaments disclosed herein may be parenterally administered as a solution of the biologically active agent suspended in an acceptable carrier, for example, an aqueous carrier. A variety of aqueous carriers may be used, e.g. water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration.

For aerosol administration, medicaments disclosed herein can be supplied along with a surfactant or polymer and propellant. The surfactant or polymer must, of course, be non-toxic, and, for example,soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant or polymer may constitute 0.1%-20% by weight of the composition, for example, 0.25-5%. The balance of the composition is ordinarily propellant. A carrier can also be included, as desired, as with, e.g., lecithin for intranasal delivery.

Medicaments disclosed herein may also be administered via liposomes, which serve to target the active agent to a particular tissue, such as lymphoid tissue, or targeted selectively to cells.

Liposomes include emulsions, foams, micelles, insoluble monolayers, liquid crystals, phospholipid dispersions, lamellar layers and the like. In these preparations the composite microstructure composition is incorporated as part of a liposome, alone or in conjunction with a molecule that binds to or with other therapeutic or immunogenic compositions.

As described above, the biologically active material can be formulated into a solid dosage form (e.g., for oral or suppository administration), together with the grinding matrix or at least a portion of it. In this case there may be little or no need to add stabilizing agents since the grinding matrix may effectively act as a solid-state stabilizer.

However, if the biologically active material is to be utilized in a liquid (or gaseous) suspension, the particles comprising the biologically active material may require further stabilization once the solid carrier has been substantially removed to ensure the elimination, or at least minimisation of particle agglomeration.

### Therapeutic uses

Therapeutic uses of the medicaments disclosed herein include pain relief, anti-inflammatory, migraine, asthma, and other disorders that require the active agent to be administered with a high bioavailability.

One of the main areas when rapid bioavailability of a biologically active material is required is in the relief of pain. The minor analgesics, such as cyclooxgenase inhibitors (aspirin related drugs) may be prepared as the medicaments disclosed herein.

Medicaments disclosed herein may also be used for treatment of eye disorders. That is, the biologically active material may be formulated for administration on the eye as an aqueous suspension in physiological saline, or a gel. In addition, the biologically active material may be prepared in a powder form for administration via the nose for rapid central nervous system penetration.

Treatment of cardiovascular disease may also benefit from biologically active materials disclosed herein , such as treatment of angina pectoris and, in particular, molsidomine may benefit from better bioavailability.

Other therapeutic uses of the medicaments disclosed herein include treatment of hair loss, sexual dysfunction, or dermal treatment of psoriasis.

The present invention will now be described with reference to the following non-limiting Examples. The description of the Examples is in no way limiting on the preceding paragraphs of this specification, but is provided for exemplification of the methods and compositions of the invention.

### Examples

It will be apparent to persons skilled in the milling and pharmaceutical arts that numerous enhancements and modifications can be made to the above described processes without departing from the basic inventive concepts. For example, in some applications the biologically active material may be pretreated and supplied to the process in the pretreated form. Furthermore, the following Examples are provided for illustrative purposes only, and are not intended to limit the scope of the processes or compositions disclosed herein.

### The following materials were used in the examples

Active pharmaceutical ingredients were sourced from commercial suppliers, excipients from either commercial suppliers such as Sigma-Aldrich or retailers, while food ingredients were sourced from retailers.

### The following mills were used for the grinding experiments

### Spex-type Mill:

Small scale milling experiments were conducted using a vibratory Spex 8000D mixer/mill. Twelve 3/8" stainless steel balls were used as the grinding media. The powder charge and grinding media were loaded into a hardened steel vial with an internal volume of approximately 75 mL. Following milling, the milled material was discharged from the vial and sieved to remove grinding media.

### Attritor-type Mill:

Small scale attritor milling experiments were performed using a 1 HD Union Process attritor mill with a 110 mL grinding chamber. The grinding media consisted of 330g 5/16" stainless steel balls. The mill was loaded through the loading port, with dry materials added initially, followed by the grinding media. The milling process was conducted with the jacket cooled at 10-20°C and the shaft rotating at 500 rpm. Upon completion of milling, the milled material was discharged from the mill and sieved to remove the grinding media.

Medium scale attritor milling experiments were performed using a 1 HD Union Process attritor mill with a 1 L grinding chamber or a 1S Union Process attritor mill with a 750 mL grinding chamber. The grinding media consisted of 3 kg of 5/16" stainless steel balls or 1.5 kg of 3/8" stainless steel balls for the 1S attritor. The 1HD mill was loaded through the loading port, with dry materials added initially, followed by the grinding media, while the grinding media was added initially, followed by the dry materials in the 1S attritor mill. The milling process was conducted with the jacket cooled at 10-20°C with the shaft rotating at 350 rpm in the 1 HD attritor or 550 rpm in the 1S attritor. Upon completion of milling, the milled material was discharged from the mill and sieved to remove the grinding media.

Medium to large scale attritor milling experiments were performed using a 1S Union Process attritor mill with a ½ gallon grinding chamber. The grinding media consisted of 7 kg of 3/8" stainless steel balls. The mill was loaded through the loading port, with the grinding media added initially, followed by the dry powders. The milling process was conducted with the jacket cooled at 18°C and the shaft rotating at 550-555 rpm. Upon completion of milling, the milled powder was discharged from the mill through the bottom discharge port at 77rpm for 5min.

### Simoloyer Mill

Medium scale milling experiments were performed in a Simoloyer CM01 (ZOZ GmbH, Germany) with a 2 L milling chamber. The grinding media consisted of 2.5 kg stainless steel media with a diameter of 5 mm. the media was loaded though the loading port followed by the dry materials. The milling vessel was cooled using water at a temperature of about 18°C. The mill speed was operated in cycle mode: at 1300 rpm for two minutes and at 500 rpm for 0.5 min and so forth. Upon completion of the milling the media was discharged from the mill using a grated valve to retain the grinding media.

### Hicom Mill

Millings performed in a nutating Hicom mill utilized 14kg of stainless steel 0.25" grinding media together with a powder charge of 480g. The mill was loaded by pre-mixing media and powder, then adding the mixture to the grinding chamber through the loading port at the top of the mill. The milling was done at 1000rpm and the mill discharged by inverting the mill and emptying through the loading port. The recovered material was sieved to separate the grinding media from the powder.

Variations to the milling conditions set out above are indicated in the variations column in the data tables. The key to these variations is shown in Table A.

### Particle size measurement:

The particle size distribution (PSD) was determined using a Malvern Mastersizer 2000 fitted with a Malvern Hydro 2000S pump unit. Measurement settings used: Measurement Time: 12 seconds, Measurement cycles: 3. Final result generated by averaging the 3 measurements. Samples were prepared by adding 200mg of milled material to 5.0mL of 1% PVP in 10mM hydrochloric acid (HCl), vortexing for 1 min and then sonicating. From this suspension enough was added into the dispersant (10mM HCl) to attain a desired obscuration level. If necessary an extra 1-2 minutes of sonication was applied using the internal sonication probe in the measurement cell. The refractive index of the active ingredient to be measured was in the range of 1.49-1.73. Any variations to this general method are summarized in Table B.

**Table A. Variations to milling conditions. Only conditions reported in the table have changed as compared to conditions reported above.**

| Variation # | Mill type | Milling Speed (rpm) | Media size (inch) | Media Mass (kg) | Offload spped (rpm) |
|---|---|---|---|---|---|
| A | 1HD 1L | | 0.25 | | |
| B | 1S 0.5gal | | | 5 | |
| C | 1S 0.5gal | | | 4 | |
| D | 1S 0.5gal | 500 | | | |
| E | 1S 0.5gal | 550-555 | | | |
| F | 1S 1.5gal | 316-318 | | 21 | |
| G | 1S 1.5gal | 500 | | 21 | |
| H | 1S 1.5gal | 355 | | 21 | |
| I | 1S 1.5gal | 355 | | 18 | |
| J | 1S 1.5gal | | | 21 | |
| K | 1S 1.5gal | | | 18.4 | |
| L | 1S 1.5gal | 400 | | | |
| M | 1S 1.5gal | | | 21 | 57 |
| N | 1S 1.5gal | | | | 57 |
| O | 1S 0.5gal | 400 | | | 400 |
| P | 1S 0.5gal | 500 | | | 350 |
| Q | HICOM | | 1/8 | | |
| R | HICOM | | | 11.7 | |

**Table B. Variations to particle size measurement conditions.**

| Variation # | Sample Dispersant | Measurement Dispersant | Addition Method |
|---|---|---|---|
| 1 | | 0.1%PVP in DI water | Powder addition |
| 2 | 0.2% Pluronic L81 in DI water | DI water | |
| 3 | | Saturated glyphosate in DI water | Powder addition |
| 4 | | Saturated glyphosate in DI water | Powder addition |
| 5 | 1%PVP in DI water | DI water | |
| 6 | | DI water | Powder addition |
| 7 | 1%PVP in DI water | Saturated creatine in DI water | |
| 8 | 1%PVP in DI water | 10mM HCl | |
| 9 | 0.2% Pluronic L81 in DI water | Acidified with 1M HCl | |
| 10 | 1%PVP in DI water | 0.1%PVP in DI water | |
| 11 | 1%PVP in DI water | 1%PVP in DI water | |
| 12 | | | Filtered before PSD measurement |

### Abbreviations:

HCl: Hydrochloric acid
Nap: Naproxen acid
PSD: Particles size distribution
PVP: Polyvinyl pyrrolidone
RI: Refractive index
Rpm: Revolutions per minute
SLS: Sodium lauryl sulphate
SSB: Stainless Steel Balls
XRD: X-Ray Diffraction

Other abbreviations used in the data tables are listed below in Table C (for actives), Table D (for matrices) and Table E (for surfactants). In the data tables single letter with example number abbreviations have been used to identify specific sample numbers within the table. The data tables shown in the figures the use of surfactant , matrix are interchangeable and do not necessarily define the nature of that material.

**Table C. Abbreviations used for active pharmaceutical ingredients.**

| **API Name** | **Abbreviation** |
|---|---|
| 2,4-Dichlorophenoxyacetic acid | 2,4D |
| Anthraquinone | ANT |
| Celecoxib | CEL |
| Cilostazol | CIL |
| Ciprofloxacin | CIP |
| Creatine Monohydrate | CRM |
| Cyclosporin A | CYA |
| Diclofenac Acid | DIC |
| Glyphosate | GLY |
| Halusulfuron | HAL |
| Indomethacin | IND |
| Mancozeb | MAN |
| Meloxicam | MEL |
| Metaxalone | MTX |
| Metsulfuron | MET |
| Naproxen Acid | NAA |
| Naproxen Sodium | NAS |
| Progesterone | PRO |
| Salbutamol | SAL |
| Sulfur | SUL |
| Tribenuran | TRI |
| **FOOD** | |
| Apricot kernel | APR |
| Cinnamon Ground | CNG |
| Cinnamon Quills | CNQ |
| Cocoa Nibs | CON |
| Cocoa Powder | COP |
| Coffee Beans | COF |
| Cloves | CLO |
| Dehydrated Peas | PEA |
| Dehydrated Beans | BEA |
| Fenegreek | FEN |
| Goji Berry | GOJ |
| Green Tea | GTE |
| Ground Ginger | GIN |
| Lavender | LAV |
| Linseed | LIN |
| Mangosteen | MST |
| Raspberry Leaf | RAS |
| Turmeric | TUR |

**Table D. Abbreviations used for excipients.**

| **Matrix Name** | **Abbreviation** |
|---|---|
| Calcium Carbonate | CAC |
| Full Cream Milk Powder | FCM |
| Glucose | GLU |
| Lactose Anhydrous | LAA |
| Lactose Monohydrate | LAC |
| Lactose Monohydrate Food Grade | LFG |
| Malic Acid | MAA |
| Maltitol | MAL |
| Mannitol | MAN |
| Skimmed Milk Powder | SMP |
| Sodium Bicarbonate | SB |
| Sodium Chloride | SC |
| Sorbitol | SOR |
| Sucrose | SUC |
| Tartaric Acid | TA |
| TriSodium Citrate Dihydrate | TCD |
| Whey Powder | WP |
| Xylitol | XYL |

**Table E. Abbreviations used for surfactants**

| **Surfactant Name** | **Abbreviation** |
|---|---|
| Aerosil R972 Silica | AS |
| Benzalkonium Chloride | BC |
| Brij700 | B700 |
| Brij76 | B76 |
| Cremophor EL | CEL |
| Cremophor RH-40 | C40 |
| Dehscofix 920 | D920 |
| Docusate Sodium | DS |
| Kollidon 25 | K25 |
| Kraftsperse 1251 | K1251 |
| Lecithin | LEC |
| Poloxamer 188 | P188 |
| Microcrystalline Cellulose | MCC |
| Poloxamer 407 | P407 |
| Polyethylene Glycol 3000 | P3000 |
| Polyethylene Glycol 8000 | P8000 |
| Polyoxyethylene 40 Stearate | P40S |
| Polyvinyl Pyrrolidone (Kollidon 30) | PVP |
| Primellose | PML |
| Primojel | PRI |
| Sodium Deoxycholate | SDC |
| Sodium Dodecyl Sulphate | SDS |
| Sodium Dodecylbenzenesulphonic Acid | SDA |
| Sodium N-Lauroyl Sarcosine | SNS |
| Sodium Octadecyl Sulphate | SOS |
| Sodium Pentane Sulphonate | SPS |
| Soluplus HS15 | SOL |
| Teric 305 | T305 |
| Tersperse 2700 | T2700 |
| Terwet 1221 | T1221 |
| Terwet 3785 | T3785 |
| Tween 80 | T80 |

### Example 1. High Volume Fraction Milling

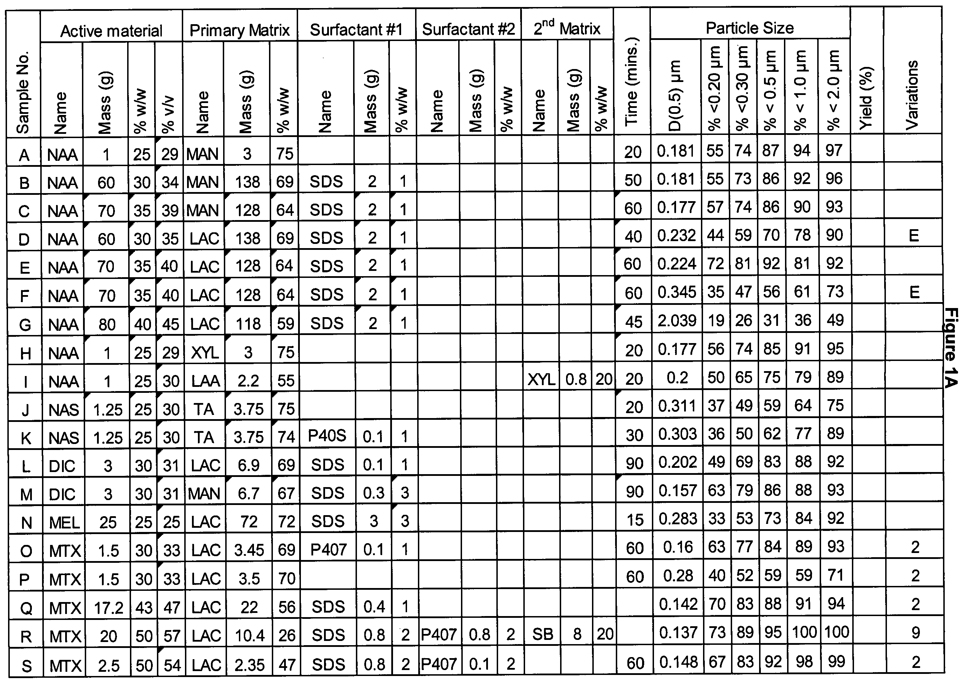

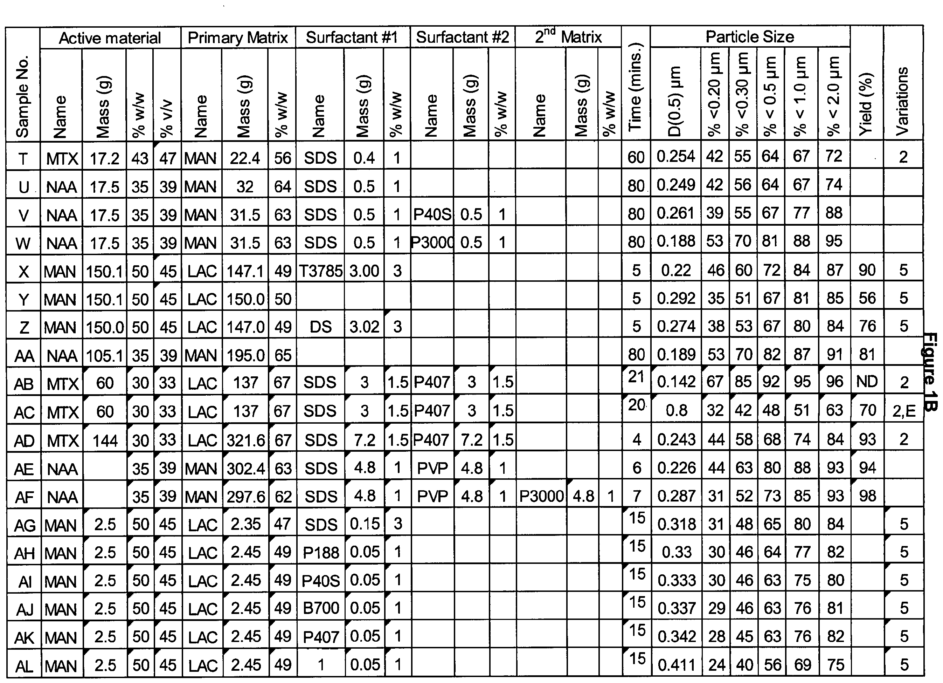

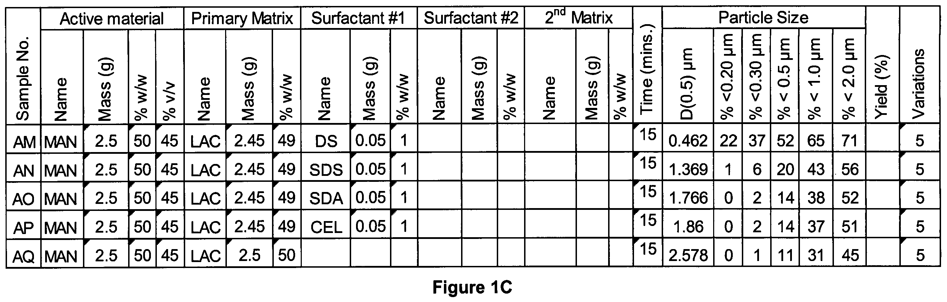

A range of actives, matrices and surfactants in a variety of combinations were milled using a variety of mills. The details of these millings are shown in Figure 1A-C together with the particle size distributions of actives that were milled.

Figure 1A-C shows that a variety of actives can be milled at high volume fraction (v/v%≥25%) and produce nanoparticles. Milling at high volume fraction can be achieved in a variety of mills as demonstrated by Samples A, H-L, O, P, S, AG-AQ, milled in SPEX mill; Samples B, D, X-AC, milled in 1/2gallon 1S attritor mill; Samples C, E, G, milled in Simoloyer horizontal attritor mill; Sample M, milled in 110mL HD01 attritor mill; Sample N, milled in 1L HD01 attritor mill; Samples Q, R, T-W, milled in 750ml 1S attritor mill and Samples AD-AF, milled in HICOM mill.

### Example 2: Naproxen

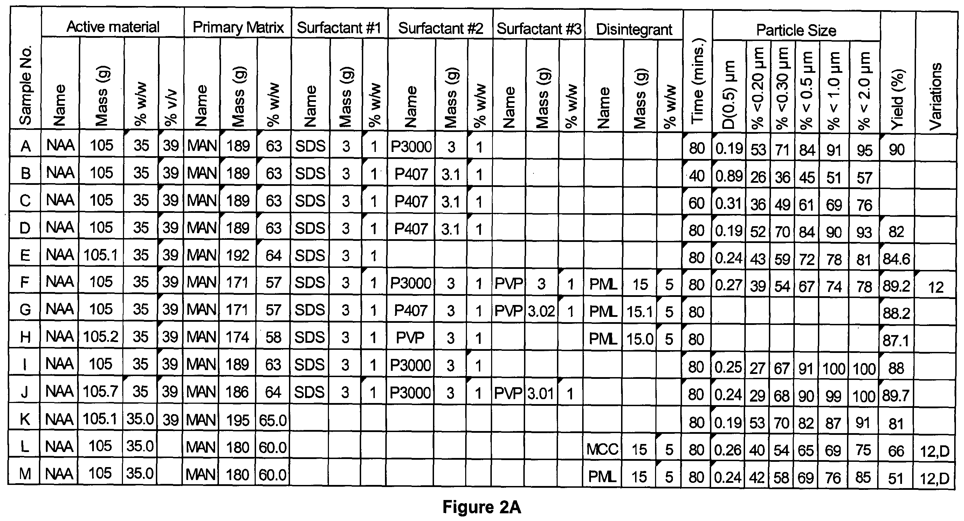

Naproxen was milled in mannitol with a range of surfactants using the 1/2 Gallon 1S mill. The details of these millings are shown in Figures 2A together with the particle size distributions of actives that were milled.

Naproxen acid milled in Mannitol with a surfactant (Sample A, D-J in Figure 2A) leads to higher yields, as compared to Naproxen acid milled in Mannitol without surfactant (Sample K, Figure 2A). Naproxen acid milled in Mannitol and either microcrystalline cellulose or the disintegrant primellose (sample L or M, Figure 2A) leads to small particle size with D(0.5) around 0.25 in both cases.

### Example 3 Filtration

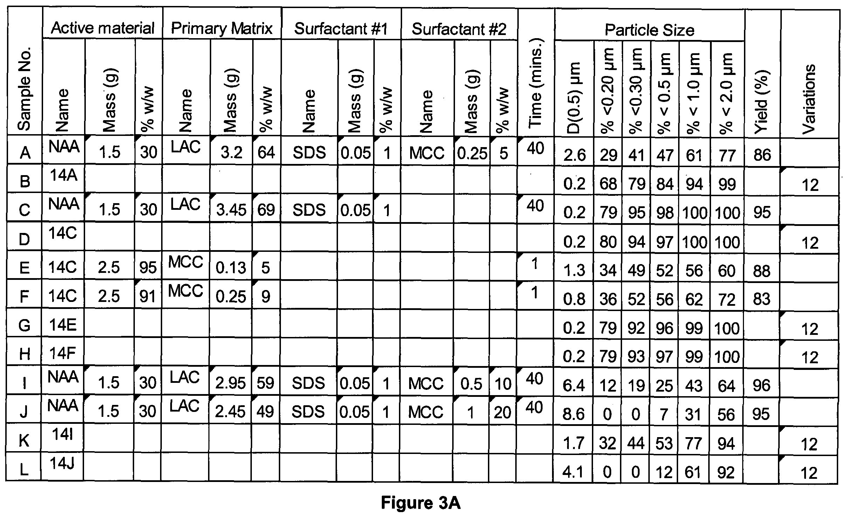

Some matrices, milling aids or facilitating agents are not water soluble. Examples of these are microcrystalline cellulose and disintegrants such as croscarmellose and sodium starch glycolate. In order to more easily characterise the particle size of the active after milling with these materials filtration methods can be used to remove them allowing a characterisation of the active. In the following examples naproxen was milled with lactose monohydrate and microcrystalline cellulose (MCC). The particle size was characterised before and after filtration and the ability of the filters to let through the naproxen was demonstrated using HPLC assays. The milling details and the particle size are shown in Figure 3a. Note in this table the particle size with milling details is unfiltered. The particle size in the rows with no milling details is after filtration. The sample that was filtered is indicated in the Active material section. The HPLC assays were performed by taking samples before and after filtration through 10 micron poroplast filters. The samples taken were diluted to give a nominal concentration of 100 µg/ml. The HPLC assay data is shown in Table 3

Sample A was milled with 5% MCC. Before filtration the D50 was 2.5 µm, after filtration (sample B) the D50 was 183 nm. When sample B was assayed the concentration was 94 µg/ml indicating that filtration process retained little naproxen. A second milling (sample C) was undertaken without MCC. The D50 was 160nm as would be expected. After filtration (sample D) the particle size was unchanged indicating that if the filtration process did remove any naproxen then it was removed in an even way. Some of sample C was then milled with MCC for 1 minute. This is long enough to incorporate the MCC into the powder but not long enough to affect the particle size distribution. Two millings were undertaken. Sample E incorporated 5 % w/w MCC into the powder and Sample F 9 % w/w. After incorporation of the MCC the particle size increased dramatically. These samples where then filtered (Sample E and F) and the size remeasured. After filtration the particle size is the same as Sample C which was the starting material. The assay of samples EH indicates that filtration did not remove any naproxen of any significance. The combination of particle size and assay data clearly shows that material such as MCC can easily and successfully be removed allowing the true particle size of the active to be measured.

Samples I and J were millings conducted with 10 and 20 % w/w MCC. The particle size post filtration is show as sample K and L. Again the filtration has delivered a reduction in particle size due to the removal of the MCC component. And again the HPLC assay of sample I-L shows little naproxen was lost during filtration.

This data also demonstrates that MCC can successfully be used as co matrix.

**Table 3: The HPLC assay of naproxen before and after filtration of samples.**

| Sample No. | HPLC Assay (µg/ml) |
|---|---|
| B | 94 |
| D | 93 |
| E | 99 |
| F | 96 |
| G | 98 |
| H | 97 |
| I | 94 |
| J | 89 |
| K | 91 |
| L | 84 |

### Example 4: Dissolution of Naproxen Nanoformulation Capsules.

### Example 4(a) Manufacture of Naproxen (200 mg) Nanoformulation Capsules.

Nine sublots of naproxen nanoformulation milled powder were combined, roller compacted, processed in a Quadro® Comil®, and encapsulated. For each milling sublot, 334 g of naproxen, 599 g of mannitol, 9.55 g of povidone K30, and 9.55 g of sodium lauryl sulfate were charged into an 8-qt V blender and mixed for 10 minutes, yielding a powder of approximate composition 35% naproxen, 63% mannitol, 1% povidone K30, and 1% sodium lauryl sulfate.

The blends were then milled individually and during the milling processes, unmilled material and samples were periodically discharged and their amounts recorded. After completion of each of the individual millings, an amount of croscarmellose sodium was added to each milling. The amount of croscarmellose sodium added was based on the theoretical amount of milled powder remaining in the mill, such that the final concentration of croscarmellose sodium in the powder would be 5.38% w/w upon addition of the calculated amount. After adding the croscarmellose sodium to the attritor mill, the mill was run for 2 minutes. The milled powder of approximate final composition 33.11% naproxen, 59.61% mannitol, 0.95% sodium lauryl sulfate, 0.95% povidone K30, and 5.38% croscarmellose sodium was then discharged from the mill.

Materials obtained were combined in a 1 cu. ft V- blender and mixed for 20 min. The mixed powder was processed in a Freund Model TF-156 roller compactor (screw speed = 13.4, roll speed = 4.1, pressure = 55 kg/cm²). The powder was processed for approximately 55 min, yielding ribbons of 2.3 to 2.7 mm thickness.

The roller compacted ribbons were manually crushed and fed into the hopper of a Quadro® Comil® 197 equipped with an 1143 micron screen and 0.225 inch spacer, operating at 2000 rpm. The net yield of milled granular material was 4.183 kg.

The milled roller compacted granules were encapsulated into size 00 white opaque hard gelatin capsules using a MiniCap 100 Capsule Filling Machine equipped with size 00 change parts. The capsules were filled manually with a scraper and periodically checked for gross weight, closure integrity, and appearance. The target fill weight was 604 mg, and the average weight of an empty capsule shell was 117 mg. The filled capsules were then polished in a capsule polishing machine. The net yield of filled, polished capsules was 4,183 g (approximately 6,925 capsules).

### Example 4(b) Dissolution rate of milled naproxen

The Dissolution of milled naproxen (200 mg) capsules (see example 4a), and commercial Naprosyn® 250 mg (naproxen) tablets (Roche Pharmaceuticals®, Inc., USA) were determined using dissolution equipment set up as USP Apparatus II (paddles) with a stirrer speed of 50 rpm. The dissolution media was 900 ml of 0.3 % SLS in 0.1 M sodium phosphate buffer at pH 5. The vessel temperature was 37 °C. The capsules where weighted down with a wire sinker. Six test articles were tested and the data average for each time point. At each time point a 1 ml sample was taken from each dissolution vessel, filtered through a 0.45 µm filter and analyzed by HPLC. The data in Table 4a below reports the percent dissolved of the amount of active in each test article, for the specified time points.

**Table 4a. Dissolution Profiles of Naprosyn® Tablets 250 mg and Naproxen Nanoformulation Capsules 200 mg**

| | Percent of Label Claim Dissolved (%) | |
|---|---|---|
| Time | Naprosyn Tablets 250 mg | Naproxen Nanoformulation Capsules 200 mg |
| 0 | 0 | 0 |
| 5 | 24 | 19 |
| 10 | 40 | 53 |
| 15 | 49 | 77 |
| 20 | 55 | 90 |
| 45 | 73 | 98 |
| 60 | 79 | 99 |

The results demonstrate that the milled naproxen capsules dissolve more quickly and more completely than the commercial reference naproxen. Those of skill in the art will readily appreciate the advantages conferred by more rapid dissolution -- more active agent is available at any given time point. Put another way, an equal quantity of dissolved naproxen may be obtained with an initially smaller dosage amount of milled naproxen, as opposed to the larger initial dose required for the reference naproxen to reach to the same quantity of dissolved naproxen. Additionally, as the results make clear, the reference naproxen does not achieve complete dissolution even by the final time point, while the milled naproxen achieves greater than 90% dissolution within 20 minutes, and substantially complete dissolution by the 45 minute time point. Again, a smaller dose of milled naproxen yields a quantity of dissolved naproxen for which a larger dose of reference naproxen would be required to equal.

The roller compacted ribbons were manually crushed and fed into the hopper of a Quadro® Comil® 197 equipped with an 1143 micron screen and 0.225 inch spacer, operating at 2000 rpm. The net yield of milled granular material was 4.183 kg.

The milled roller compacted granules were encapsulated into size 00 white opaque hard gelatin capsules using a MiniCap 100 Capsule Filling Machine equipped with size 00 change parts. The capsules were filled manually with a scraper and periodically checked for gross weight, closure integrity, and appearance. The target fill weight was 604 mg, and the average weight of an empty capsule shell was 117 mg. The filled capsules were then polished in a capsule polishing machine. The net yield of filled, polished capsules was 4,183 g (approximately 6,925 capsules).

### Example 4(b) Dissolution rate of milled naproxen

The Dissolution of milled naproxen (200 mg) capsules (see example 4a), and commercial Naprosyn® 250 mg (naproxen) tablets (Roche Pharmaceuticals®, Inc., USA) were determined using dissolution equipment set up as USP Apparatus II (paddles) with a stirrer speed of 50 rpm. The dissolution media was 900 ml of 0.3 % SLS in 0.1 M sodium phosphate buffer at pH 5. The vessel temperature was 37 °C. The capsules where weighted down with a wire sinker. Six test articles were tested and the data average for each time point. At each time point a 1 ml sample was taken from each dissolution vessel, filtered through a 0.45 µm filter and analyzed by HPLC. The data in Table 4a below reports the percent dissolved of the amount of active in each test article, for the specified time points.

**Table 4a. Dissolution Profiles of Naprosyn® Tablets 250 mg and Naproxen Nanoformulation Capsules 200 mg**

| | Percent of Label Claim Dissolved (%) | |
|---|---|---|
| Time | Naprosyn Tablets 250 mg | Naproxen Nanoformulation Capsules 200 mg |
| 0 | 0 | 0 |
| 5 | 24 | 19 |
| 10 | 40 | 53 |
| 15 | 49 | 77 |
| 20 | 55 | 90 |
| 45 | 73 | 98 |
| 60 | 79 | 99 |

The results demonstrate that the milled naproxen capsules dissolve more quickly and more completely than the commercial reference naproxen. Those of skill in the art will readily appreciate the advantages conferred by more rapid dissolution -- more active agent is available at any given time point. Put another way, an equal quantity of dissolved naproxen may be obtained with an initially smaller dosage amount of milled naproxen, as opposed to the larger initial dose required for the reference naproxen to reach to the same quantity of dissolved naproxen. Additionally, as the results make clear, the reference naproxen does not achieve complete dissolution even by the final time point, while the milled naproxen achieves greater than 90% dissolution within 20 minutes, and substantially complete dissolution by the 45 minute time point. Again, a smaller dose of milled naproxen yields a quantity of dissolved naproxen for which a larger dose of reference naproxen would be required to equal.

## Claims

1. A method for producing a composition, comprising the steps of:
dry milling a solid biologically active material, a milling aid or combination of milling aids, and a millable grinding matrix in a mill comprising a plurality of milling bodies, for a time period sufficient to produce particles of the biologically active material dispersed in an at least partially milled grinding material, wherein the composition produced by said method comprises particles of the biologically active compound at or above a volume fraction of 25 v/v%, wherein the particles have a median particle size, determined on a particle volume basis, equal or less than 800 nm.

2. The method of claim 1, wherein the particles have a median particle size, determined on a particle volume basis, equal or less than a size selected from the group consisting of: 700nm, 600nm, 500nm, 400 nm, 300nm, 200nm and 100 nm.

3. The method of claim 1, wherein the milling aid is selected from the group consisting of: colloidal silica, a solid or semi solid surfactant, a liquid surfactant, a surfactant that can be manufactured into a solid or semisolid, a polymer, a stearic acid and derivatives thereof.

4. The method of claim 3, wherein the surfactant is selected from the group consisting of: polyoxyethylene alkyl ethers, polyoxyethylene stearates, poloxamers, sarcosine based surfactants, polysorbates, alkyl sulfates and other sulfate surfactants, ethoxylated castor oil, polyvinylpyrrolidones, deoxycholate based surfactants, trimethyl ammonium based surfactants, lecithin and other phospholipids and bile salts.

5. The method of claim 3 wherein the milling aid is sodium lauryl sulfate.

6. The method of claim 1 wherein the millable grinding matrix is lactose anhydrous or lactose monohydrate.

7. The method of claim 1 wherein the millable grinding matrix is mannitol.

## Patentansprüche

1. Verfahren zur Herstellung einer Zusammensetzung, das die folgenden Schritte umfasst:
Trockenmahlen eines festen, biologisch aktiven Materials, eines Mahlhilfsmittels oder einer Kombination von Mahlhilfsmitteln und einer mahlbaren Zerkleinerungsmatrix in einer Mühle, die eine Mehrzahl von Mahlkörpern umfasst, für einen Zeitraum, der ausreicht, dass Partikel des biologisch aktiven Materials, dispergiert in einem zumindest zum Teil gemahlenen Zerkleinerungsmaterial, hergestellt werden, wobei die durch dieses Verfahren hergestellte Zusammensetzung Partikel der biologisch aktiven Verbindung in oder über einem Volumenanteil von 25% v/v umfasst, wobei die Partikel eine, bezogen auf das Partikelvolumen bestimmte, mittlere Partikelgröße von gleich oder kleiner als 800 nm besitzen.

2. Verfahren nach Anspruch 1, wobei die Partikel eine, bezogen auf das Partikelvolumen bestimmte, mittlere Partikelgröße gleich einer oder kleiner als eine Größe besitzen, die aus der Gruppe ausgewählt ist, bestehend aus: 700 nm, 600 nm, 500 nm, 400 nm, 300 nm, 200 nm und 100 nm.

3. Verfahren nach Anspruch 1, wobei das Mahlhilfsmittel aus der Gruppe ausgewählt ist, bestehend aus: kolloidalem Siliziumdioxid, einem festen oder halbfesten Tensid, einem flüssigen Tensid, einem Tensid, das zu einem Feststoff oder Halbfeststoff verarbeitet werden kann, einem Polymer, einer Stearinsäure und Derivaten davon.

4. Verfahren nach Anspruch 3, wobei das Tensid aus der Gruppe ausgewählt ist, bestehend aus: Polyoxyethylenalkylethern, Polyoxyethylenstearaten, Poloxameren, Tensiden auf Sarkosin-Basis, Polysorbaten, Alkylsulfaten und anderen Sulfat-Tensiden, ethoxyliertem Rizinusöl, Polyvinylpyrrolidonen, Tensiden auf Desoxycholat-Basis, Tensiden auf Trimethylammonium-Basis, Lecithin und anderen Phospholipiden und Gallensalzen.

5. Verfahren nach Anspruch 3, wobei das Mahlhilfsmittel Natriumlaurylsulfat ist.

6. Verfahren nach Anspruch 1, wobei die mahlbare Zerkleinerungsmatrix Lactose wasserfrei oder Lactose-Monohydrat ist.

7. Verfahren nach Anspruch 1, wobei die mahlbare Zerkleinerungsmatrix Mannit ist.

## Revendications

1. Procédé de production d'une composition, comprenant les étapes de : broyage à sec d'un matériau biologiquement actif solide, un adjuvant de broyage ou une combinaison d'adjuvants de broyage, et une matrice de broyage broyable dans un broyeur comprenant une pluralité de corps de broyage, pendant une durée suffisante pour produire des particules du matériau biologiquement actif dispersées dans un matériau de broyage au moins partiellement broyé, dans lequel la composition produite par ledit procédé comprend des particules du composé biologiquement actif à ou au-dessus d'une fraction volumique de 25 % v/v, dans lequel les particules ont une taille de particule médiane, déterminée sur une base de volume de particule, égale ou inférieure à 800 nm.

2. Procédé de la revendication 1, dans lequel les particules ont une taille de particule médiane, déterminée sur une base de volume de particules, égale ou inférieure à une taille choisie dans le groupe constitué de : 700 nm, 600 nm, 500 nm, 400 nm, 300 nm, 200 nm et 100 nm.

3. Procédé de la revendication 1, dans lequel l'adjuvant de broyage est choisi dans le groupe constitué de : une silice colloïdale, un tensioactif solide ou semi-solide, un tensioactif liquide, un tensioactif qui peut être fabriqué dans un solide ou semi-solide, un polymère, un acide stéarique et des dérivés de ceux-ci.

4. Procédé de la revendication 3, dans lequel le tensioactif est choisi dans le groupe constitué de : éthers d'alkyle de polyoxyéthylène, stéarates de polyoxyéthylène, poloxamères, tensioactifs à base de sarcosine, polysorbates, sulfates d'alkyle et d'autres tensioactifs à base de sulfate, huile de ricin éthoxylée, polyvinylpyrrolidones, tensioactifs à base de désoxycholate, tensioactifs à base de triméthylammonium, lécithine et d'autre phospholipides et sels biliaires.

5. Procédé de la revendication 3 dans lequel l'adjuvant de broyage est le laurylsulfate de sodium.

6. Procédé de la revendication 1 dans lequel la matrice de broyage broyable est le lactose anhydre ou le lactose monohydraté.

7. Procédé de la revendication 1 dans lequel la matrice de broyage broyable est le mannitol.
